# EUROPEAN PATENT SPECIFICATION

(11) **EP 1 682 024 B1**
(45) Date of publication and mention of the grant of the patent: **07.11.2012**
(21) Application number: 04784249.7
(22) Date of filing: 16.09.2004
(51) Int. Cl.: A61M 25/01

(54) **USER INTERFACE FOR REMOTE CONTROL OF MEDICAL DEVICES**
ANWENDERSCHNITTSTELLE FÜR DIE FERNSTEUERUNG VON MEDIZINPRODUKTEN
INTERFACE UTILISATEUR POUR LA COMMANDE A DISTANCE DE DISPOSITIFS MEDICAUX

(30) Priority: 16.09.2003 US 503684 P
(43) Date of publication of application: 26.07.2006
(60) Divisional application: 09172578.8
(73) Proprietor: STEREOTAXIS, INC., St. Louis, MO 63108 (US)
(72) Inventor: VISWANATHAN, Raju, St.Louis MO 63108 (US); BLUME, Walter, M., Webster Groves, MO 63119 (US); RAUCH, John, St. Louis, MO 63109 (US); GARIBALDI, Jeffrey, M., St. Louis, MO 63129 (US)
(74) Representative: Hedges, Martin Nicholas
(86) International application number: PCT/US2004/030318
(87) International publication number: WO 2005/029258

(56) References cited:
- WO-A-00/07641
- US-A- 5 125 888
- US-A- 6 014 580
- US-A- 6 118 845
- US-B1- 6 272 370
- US-B1- 6 298 257

## Description

This invention relates to the remote navigation of medical devices in a patient's body, and in particular to a user interface for controlling a remote navigation system.

Advances in technology have resulted in systems that allow a physician or other medical professional to remotely control the orientation of the distal of a medical device. It is now fairly routine to steer the distal end of a medical device inside a patient's body by manipulating controls on the proximal end of the medical device. Recently magnetic navigation systems have been developed that allow a physician to orient the distal end of a medical device using the field of an external source magnet. Other systems have been developed for the automated remote orientation of the distal end of a medial device, for example by operating magnetorestrictive or electrorestrictive elements incorporated into the medical device. However the medical device is oriented, it is still difficult for a physician to visualize the procedure site (which is out of view inside the patient's body), to select the desired direction in which to orient the distal end of the medical device and communicate the selected direction to the system in order to orient the distal end of the medical device in the selected direction.

WO 0007641 discloses a system for navigating a medical device in an operating region in which a pair of displays are provided illustrating different perspective of the operating region, a user identifying a point on each display using an input device, from which information the system is able to determine a desired three dimensional orientation of the medical device.

WO 9923946 similarly discloses a system in which a pair of screens are used to identify a point in three dimensions.

According to the present invention there is provided an interface for controlling a magnetic navigation system that applies a magnetic field in a selected direction to an operating region in a subject to magnetically orient a medical device in the operating region, the interface including: a display on which at least one image of the operating region is displayed, and an input device for identifying a point in the operating region on the at least one image on the display; and characterised by a processor for determining an application point in the operating region on a predetermined branched path through the subject's vasculature that is closest to the identified point, and causing the magnetic navigation system to apply a magnetic field at the application point, in a direction tangent to the predetermined path at the application point.

The present invention further provides a magnetic navigation system that applies a magnetic field in a selected direction to an operating region in a subject to magnetically orient a medical device in the operating region, the system comprising at least one magnet, and an interface according to the invention.

The present invention relates to an interface to facilitate the selection of the desired direction in which to orient the distal end of the medical device and to communicate the selected direction to a navigation system in order to orient the distal end of the medical device in the selected direction. While the present invention is described primarily in connection with a magnetic navigation system, the invention is not so limited, and can be used in connection with other navigation systems, such as those that can orient the distal end of a medical device with mechanical means, electrorestrictive elements, magnetorestrictive elements, or otherwise.

In order that the invention may be well understood, there will now be described some embodiments thereof, given by way of example reference being made to the accompanying drawings, in which:
Fig. 1 is a schematic diagram of an interface system according to the principles of this invention.
Fig. 2 is a schematic diagram of a possible implementation of the interface for use in controlling a magnetic surgery system;
Fig. 3 is a schematic diagram of the display of a first preferred embodiment of the interface of this invention;
Fig. 4A is a view of the display of the first preferred embodiment of the interface of this invention, showing several points on the 3-D display pane and the desired orientation arrow;
Fig. 4B is a view of the display of the first preferred embodiment of the interface of the invention, showing several points on the 3-D display pane, a current direction vector and a desired direction vector;
Fig. 4C is a view of the display of the first preferred embodiment of the interface of the invention, showing the anatomical model in the 3-D display pane, with the picture-in-picture feature turned off;
Fig. 4D is a view of the display of the first preferred embodiment of the interface of the invention, showing the bull's eye display in the 3-D display pane;

Fig. 4E is a view of the display of the first preferred embodiment of the interface of the invention, showing the bull's eye display in the picture-in-picture portion of the 3-D display pane;

Fig. 4F is a view of the display of the first preferred embodiment of the interface of the invention, showing the bull's eye display in the picture-in-picture portion of the 3-D display pane, and anatomical model in the main 3-D display with the viewpoint changed from Fig. 4E;

Fig. 4G is a view of the display of the first preferred embodiment of the interface of the invention;

Fig. 4H is a view of the display of the first preferred embodiment of the interface of the invention;

Fig. 4I is a view of the display of the first preferred embodiment of the interface of the invention;

Fig. 4J is a view of the display of the first preferred embodiment of the interface of the invention;

Fig. 5 is an enlarged view of the 3-D display pane of the first preferred embodiment of the interface of this invention;

Fig. 6A and Fig. 6B are left anterior oblique (LAO) and right anterior oblique (RAO) images of the procedure site with desired orientation arrow and visualization surface superposed thereon;

Fig. 6C is an alternate implementation of the visualization surface superposed thereon

Fig. 7 is an enlarged view of the status pane of the first preferred embodiment of the interface of this invention;

Fig. 8A is an enlarged view of the 2-D navigation pane of the first preferred embodiment of the interface of this invention;

Fig. 8B is an enlarged view of an alternate embodiment of the 2-D navigation pane of Fig. 8A;

Fig. 9 is an enlarged view of the point navigation pane of the first preferred embodiment of the interface of this invention;

Fig. 10 is an enlarged view of the vector navigation pane of the first preferred embodiment of the interface of this invention;

Fig. 11 is an enlarged view of the bull's eye navigation pane of the first preferred embodiment of the interface of this invention;

Fig. 12 is a view of an x-ray image showing the projection of the bull's eye screen thereon;

Fig. 13 is an enlarged view of the menu bar of the first preferred embodiment of the interface of this invention;

Fig. 14 is a schematic diagram of the display of a second preferred embodiment of the interface of this invention;

Fig. 15 is a view of the display of the second preferred embodiment of the interface of this invention;

Fig. 16 is a view of the display of the second preferred embodiment of the interface of this invention, showing an alternate image in pane 308;

Fig. 17 is a view of the display of the second preferred embodiment of the interface of this invention, showing the use of target navigation pane 314;

Fig. 18 is a view of the display of the second preferred embodiment of the interface of this invention, showing a possible path of a medical device;

Fig. 19 is a view of the display of the second preferred embodiment of the interface of this invention, showing an elliptical constellation of points and a possible path of a medical device to the constellation;

Fig. 20 is a view of a display of a third preferred embodiment of an interface in accordance with the principles of this invention, with two main panes;

Fig. 21 is a view of a display of a third preferred embodiment of an interface of the third embodiment, with four main panes;

Fig. 22A is a view of a display of the third preferred embodiment, with biplanar images in the main panes, showing the selection of a point in creating a predetermined branched path;

Fig. 22B is an enlarged view of the biplanar images in the main panes;

Fig. 23A is a view of a display of the third preferred embodiment, with biplanar images in the main panes, showing the completion of a predetermined branched path;

Fig. 23B is an enlarged view of the biplanar images in the main pane of Fig. 23A;

Fig. 24A is a view of a display of the third preferred embodiment, with biplanar images in the main panes, showing the specification of a navigation field at an application point;

Fig. 24B is an enlarged view of the biplanar images in the main pane of Fig. 24A;

Fig. 25A is a view of a display of the third preferred embodiment, with biplanar images in the main panes, showing the specification of a navigation field at an application point;

Fig. 25B is an enlarged view of the biplanar images in the main pane of Fig. 25A;

Fig. 26A is a view of a display of the third preferred embodiment, with biplanar images in the main panes, showing the an exemplary branched path;

Fig. 26B is an enlarged view of the biplanar images in the main pane of Fig. 26A;

Fig. 27A is an enlarged view of a pattern navigation control pane of the third preferred embodiment embodiment of this invention;

Fig. 27B is an enlarged view of the pattern navigation control pane after navigation of the medical device to the first new position in the pattern;

Fig. 27C is an enlarged view of the pattern navigation control pane as the medical device is moved from the first new position in the pattern to the second new position;

Fig. 27D is an enlarged view of the pattern navigation control pane after navigation of the medical device to the second new position in the pattern;

Fig. 28 is a view of a display of a third preferred embodiment of an interface in accordance with the principles of this invention, showing another control for specifying the direction of the magnetic field to be applied;

Fig. 29 is a view of a display of a third preferred embodiment of an interface in accordance with the principles of this invention, showing another control for specifying the direction of the magnetic field to be applied;

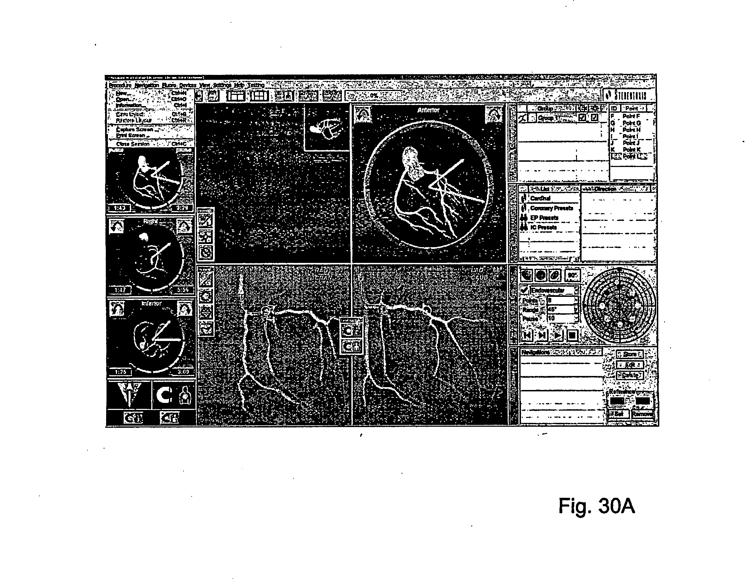

Fig. 30A is a view of a display of a third preferred embodiment of an interface in accordance with the principles of this invention, showing an orientation element;

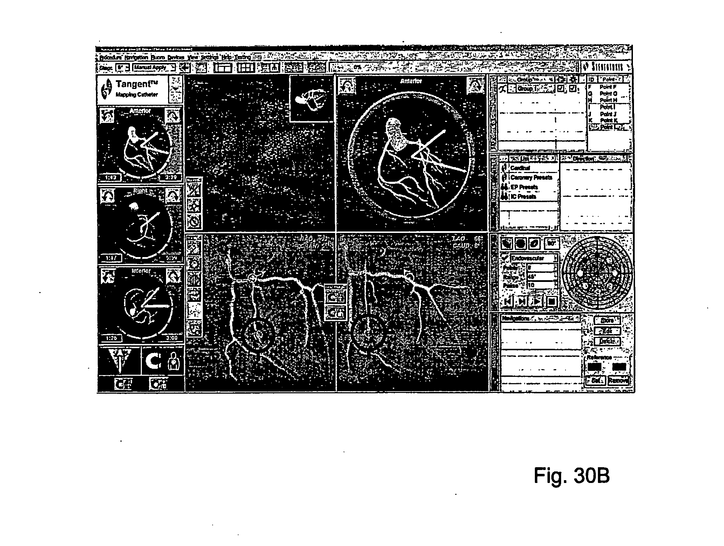

Fig. 30B is a view of a display of a third preferred embodiment of an interface in accordance with the principles of this invention, showing an orientation element; and

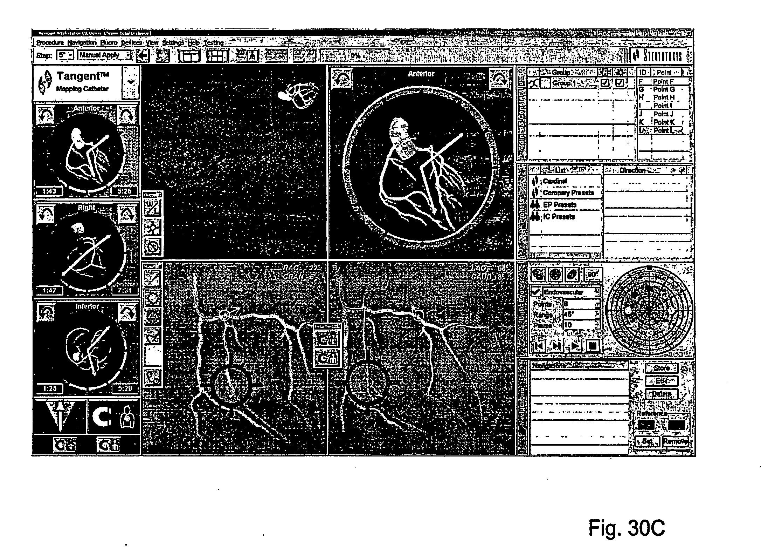

Fig. 30C is a view of a display of a third preferred embodiment of an interface in accordance with the principles of this invention, showing an orientation element.
Corresponding reference numerals indicate corresponding parts throughout the several views of the drawings.

### DETAILED DESCRIPTION OF THE INVENTION

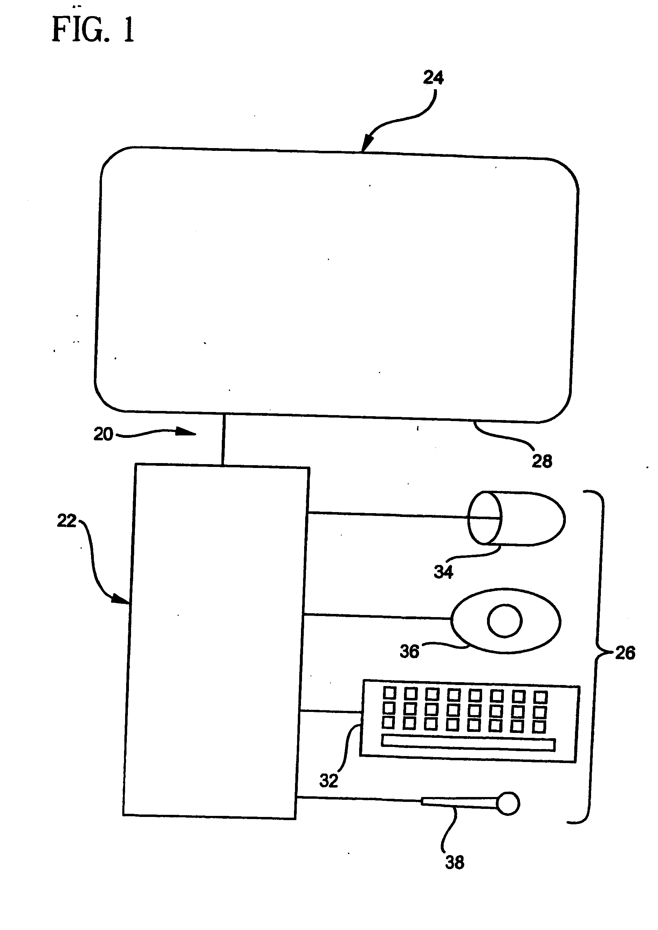

This invention relates to an interface for a navigation system for orienting the distal end of a medical device inside a patient's body. As shown in Fig. 1 the interface, indicated generally as 20, comprises a processor 22, a display 24, and an input device 26. The display 24 preferably includes at least one monitor 28, which may be a flat panel lcd display which is small, compact, and less prone to interference. The input device 26 may include a keyboard 32, a mouse 34, a track ball 36, microphone 38, or other device for controlling a cursor on the display 24.

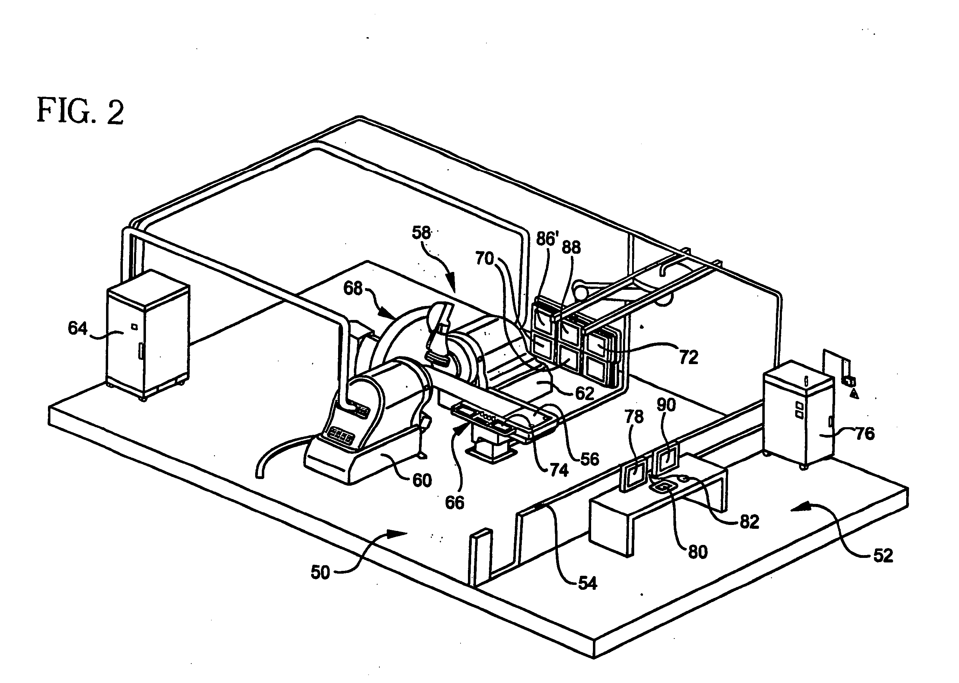

A possible implementation of an interface system is indicated in Fig. 2, in which components of the interface are distributed in the procedure room 50 where the patient is located, and a control room 52. The control room 52 is preferably adjacent the procedure room 50, and there may be a window 54 between the control room and the procedure room to permit direct observation of the patient, however the control room could be remote from the patient, and with the aid of the present interface, a physician could conduct a procedure on a patient in the procedure from a control room on a different floor, in a different building, or even in a different city.

As shown in Fig. 2, a magnetic surgery suite comprising a patient bed 56, and a magnetic navigation system 58 comprising opposed magnet units 60 and 62 on opposite sides of the patient bed operated by a processor 64 and controlled by controls 66 adjacent the patient 56. An imaging system 68, such as a x-ray imaging on a C-arm, displays images of the operating region on a monitors 70 in the procedure room 50. The interface system of the present invention provides a convenient way for a physician to operate the magnetic navigation system 58 to control the distal end of a medical device in the operating region inside the patient's body.

The interface includes a display on, for example, an lcd monitor 72, and a mouse 74 in the procedure room 50, a processor 76, a display on, for example, monitor 78, a key board 80, and a mouse 82 in the control room 54. Additional displays on monitors 86 and 88 can be provided in the procedure room 50 which integrate images from the imaging system 68 with the interface. One or more additional monitors 90 can be provided in the control room so that the images are available in the control room as well. The monitors 72 and 78 preferably display a multi-pane display.

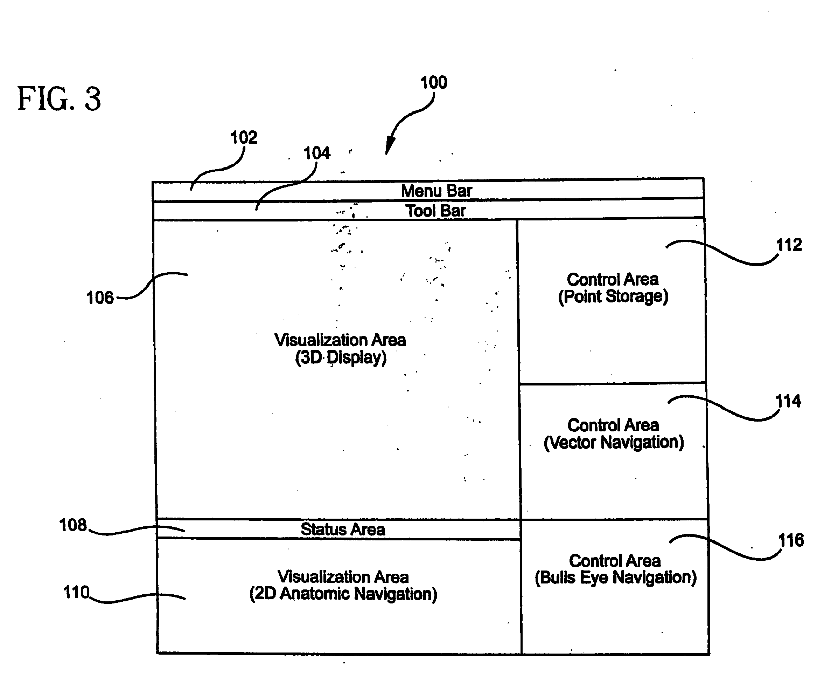

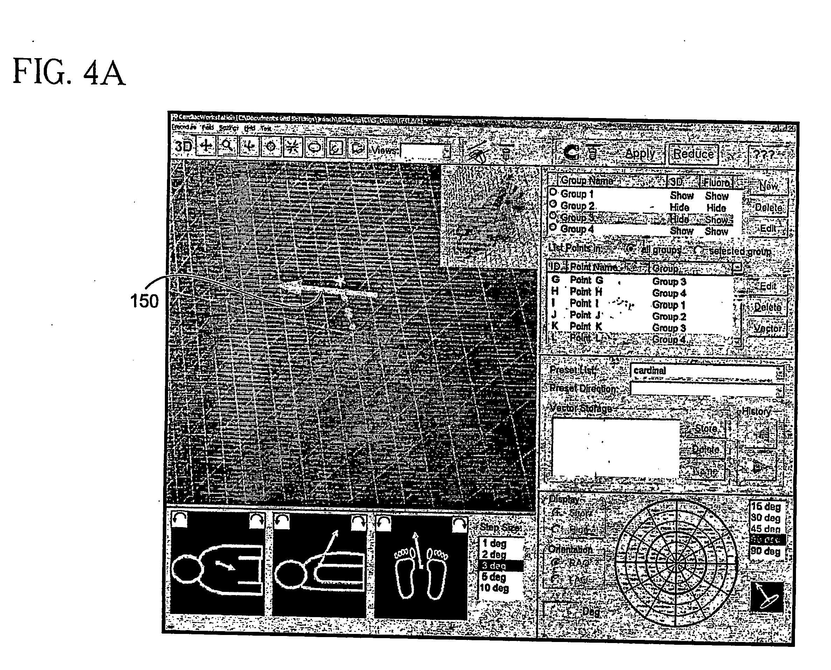

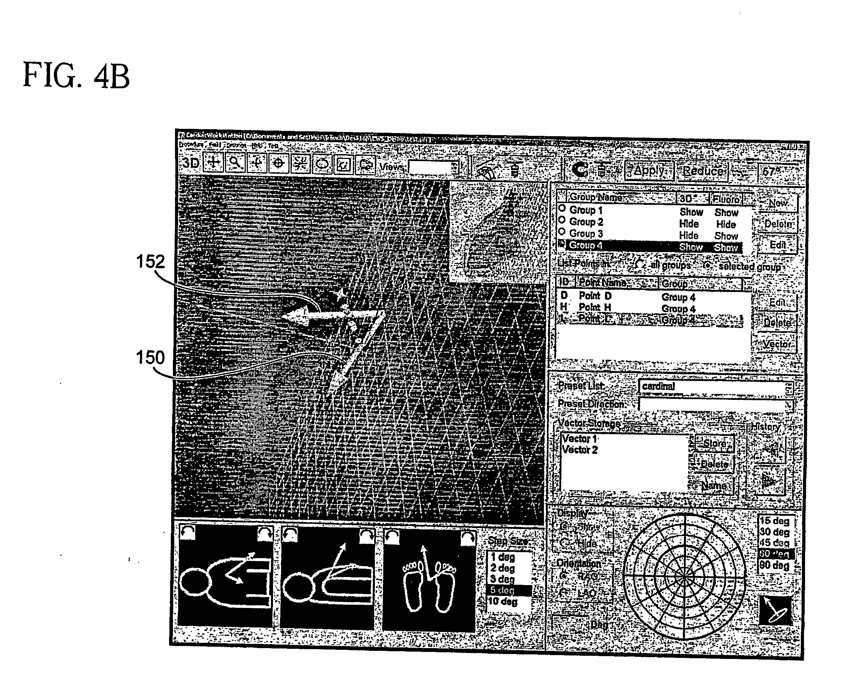

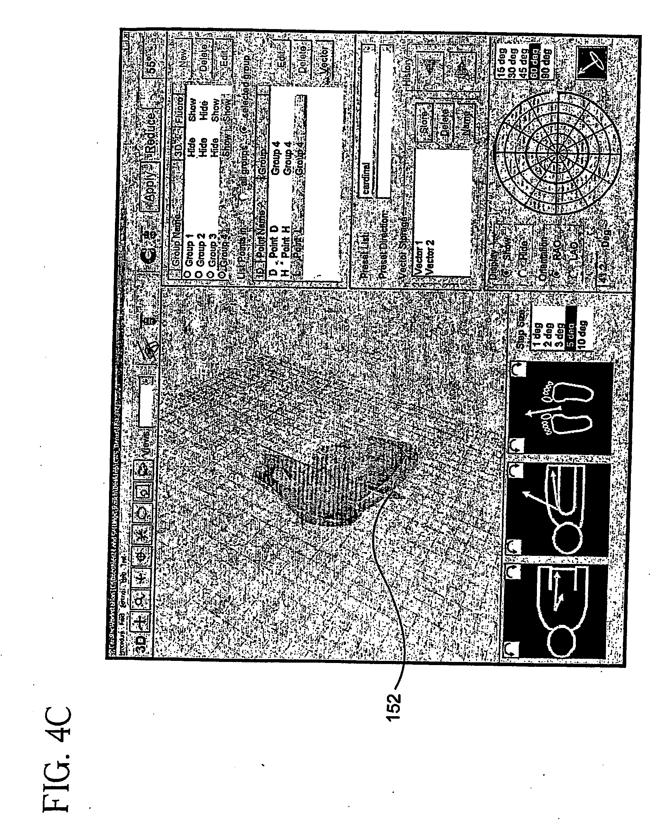

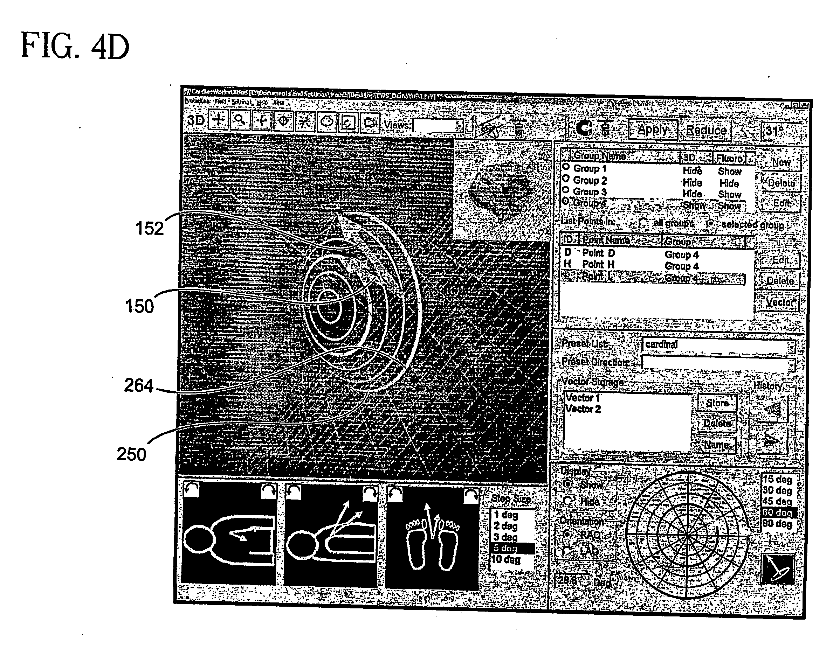

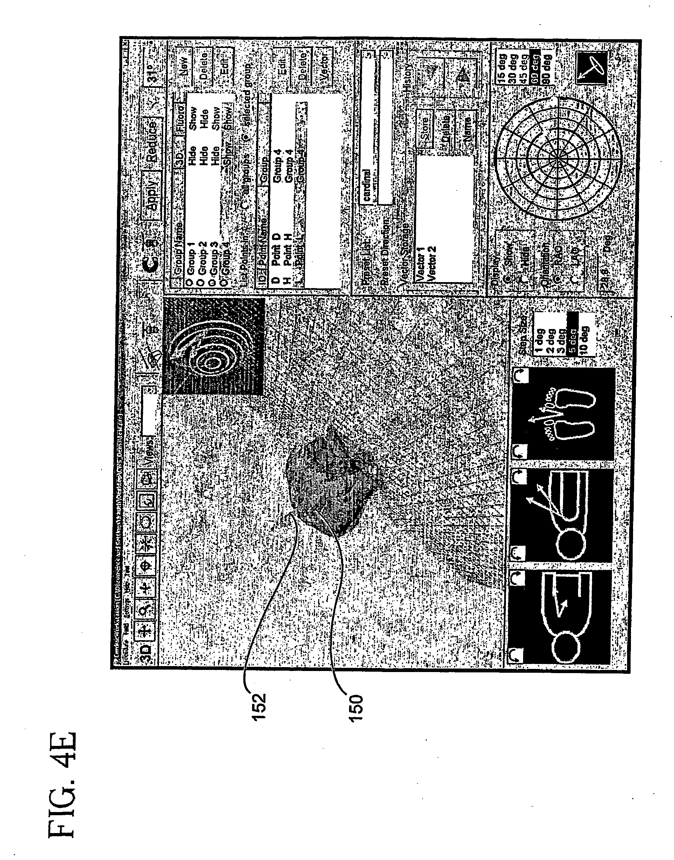

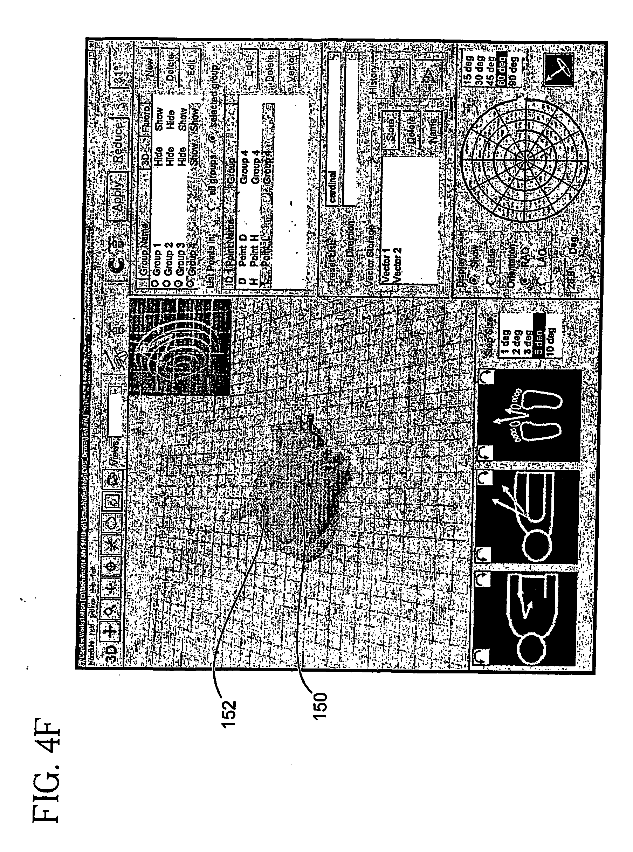

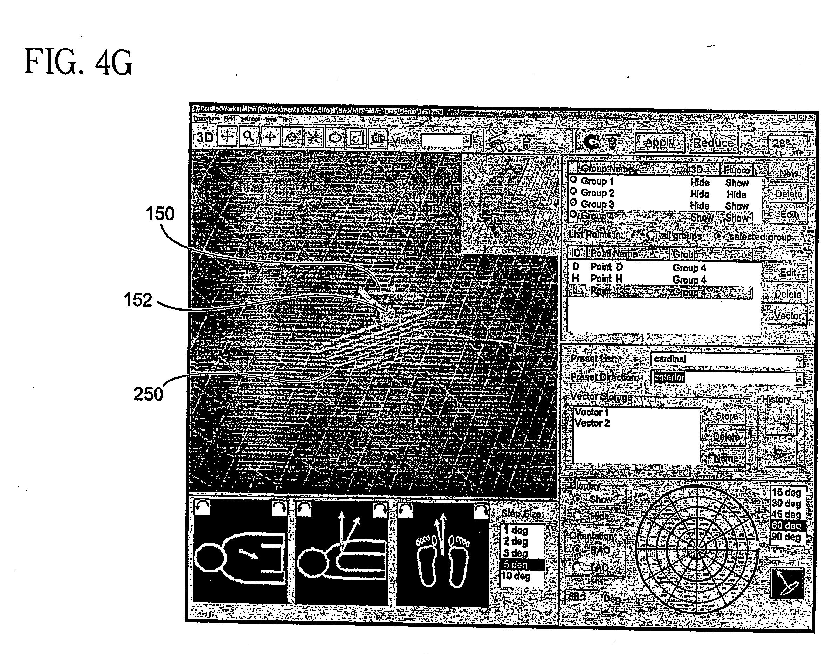

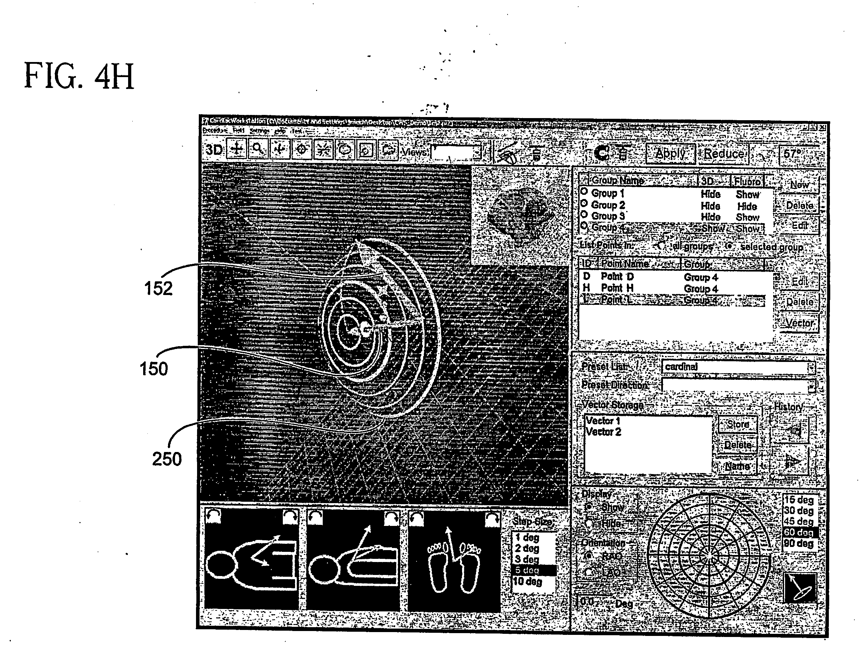

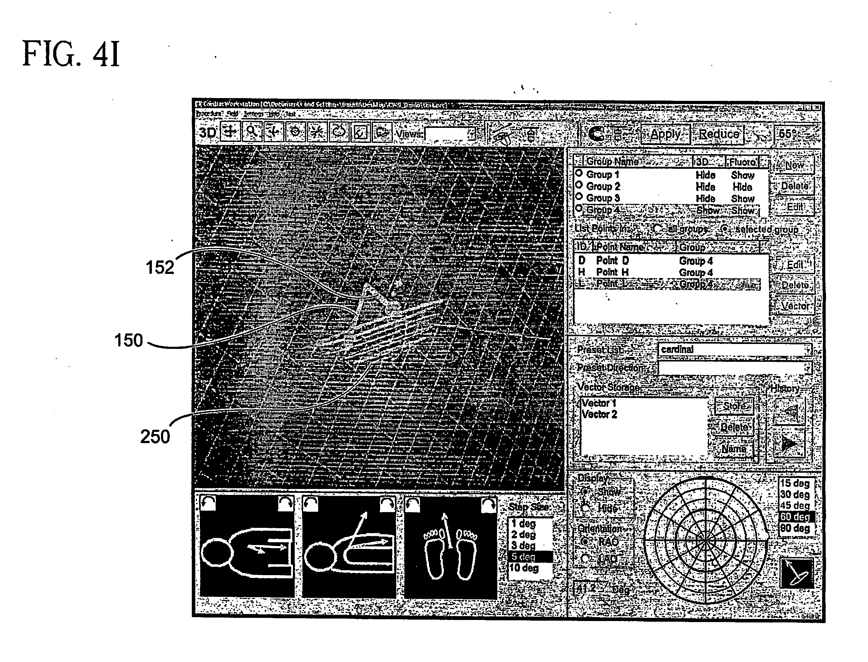

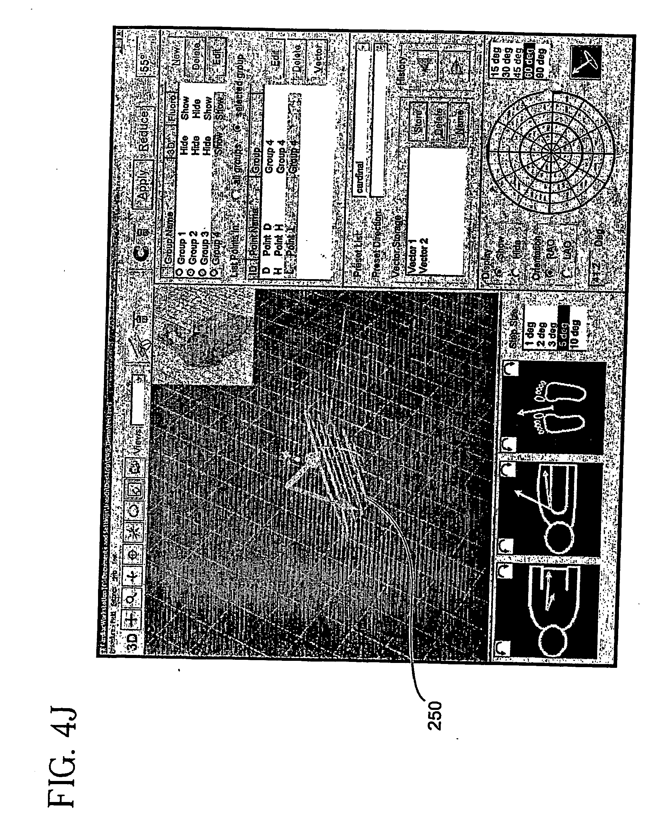

In a first preferred embodiment, as shown in Figs. 3 the display 100 on the monitors 72 and 78, includes a menu bar 102, a tool bar 104, a 3-D display pane 106, a status area 108, a 2-D anatomical control pane 110, a point navigation control pane 112, and a vector navigation control pane 114, and a bull's eye navigation control pane 116. Of course the display 100 could include additional panes or fewer panes or different panes. An example of a display in accordance with this invention is shown in Fig. 4.

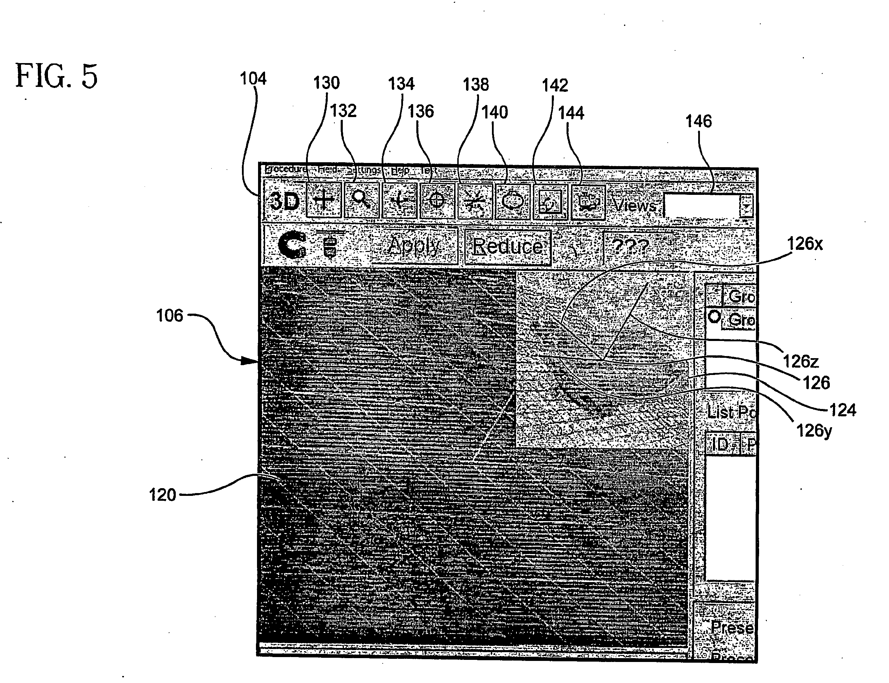

A 3-D display pane 106 in accordance with this invention is shown in Fig. 5. The display preferably includes a three-dimensional representation 120 of the patient orientation. As shown in Fig. 5 this representation 120 may be a representation of a horizontal grid corresponding to the surface of the patient support 56. Alternatively, the may be a three dimensional representation of an idealized patient, or of the patient support 56. A coordinate system 122 is optionally included in the representation to facilitate the physician's understanding of the orientation. In the first preferred embodiment, the coordinate system 122 comprises a longitudinal axis 122x, which might for example be colored blue, a horizontal axis 122y, which might for example be colored red, and a anterior-posterior axis 122z, which might, for example be colored green. The pane 106 preferably also includes a subpane 124 that displays three dimensional representation of the operating region. In this first preferred embodiment this representation is an transparent, three dimensional idealized representation of the portion of the patient's body in which the procedure is taking place, e.g. a human heart as shown in Fig. 5. To facilitate the user's interpretation of the image, the image may be displayed over a horizontal backing grid. Instead of an idealized representation of the procedure site, the image could be an actual preoperative image, or a actual current image. A coordinate system 126 is optionally included in the representation to facilitate the user's understanding of the orientation. In the first preferred embodiment, the coordinate system 126 comprises a longitudinal axis 126x, parallel to the direction as axis 122x, and which may similarly be colored blue, a horizontal axis 126y, parallel to the direction of axis 122y, and which may similarly be colored red, and a anterior-posterior axis 126z, parallel to the direction of axis 122z, and which may similarly be colored green.

The tool bar 104 includes a 3D tool bar 128 with controls for controlling the 3-D display pane 106. In this first preferred embodiment, these controls include a translation button 130, a magnification button 132, a rotation button 134, a point selection button 136, a point centering button 138, a image autorotate button 140, a swap button 142, and an image capture button 144. These buttons are preferably "virtual buttons" , i.e., the are elements on the display which the user can operate by pointing a cursor and clicking.

A view selection menu bar 146 is also provided on the 3D tool bar 128. The view selection menu 146 has an arrow that can be operated to drop down a menu of views to display in the pane 106. These preferably include cranial, caudal, anterior, posterior, left and right, as well as one or more user defined views. Of course other standard views could be provided depending upon the procedures for which the interface is used.

The translation button 130 can be actuated to enter the viewpoint translation mode by pointing the cursor to the button and clicking. In the viewpoint translation mode, the cursor might change in appearance, for example to a shape corresponding to the icon on the button 130. In this mode the view point can be changed by grabbing the image by clicking when the cursor is on the image, and dragging the cursor to move the image and thus the viewpoint in any direction. The cursor can be moved using mouse 74 or 82. This preferably also causes a corresponding translation of the view point of the image in the subpane 124.

The magnification button 132 can be operated to enter the magnification or zoom mode by pointing the cursor to the button and clicking, for example with mouse 74 or 82. In the zoom mode the cursor might change in appearance, for example to a shape corresponding to the magnifying glass icon on the button 132. In this mode the magnification of the patient reference image 120 can be accomplished by grabbing the image by pointing the cursor and clicking, and dragging the cursor downwardly and/or to the right to increase the magnification, or upwardly or to the left to decrease the magnification. Changing the size of the patient reference image preferably also does not change the size of the procedure site reference image.

The rotation button 134 can be operated to enter the image rotation mode by pointing the cursor to the button and clicking, for example with mouse 74 or 82. In the image rotation mode the cursor might change in appearance, for example to a shape corresponding to the shape on the button 134. In this mode the image can be rotated by grabbing the image by pointing the cursor and clicking, and dragging the cursor horizontally to rotate the view point of the image about a generally vertical axis, and vertically to rotate the view point about a generally horizontal axis. Of course the image can be dragged both horizontally and vertically to rotate the axis about a diagonal axis. Rotating the patient reference image preferably also rotates the procedure site reference image, so that these two images always have the same viewpoint.

The point select button 136 can be operated to enter the point selection mode by pointing the cursor to the button and clicking, for example with mouse 74 or 82. In the point selection mode the cursor might change in appearance, for example to a shape corresponding to the shape on the button 136. In this mode a point n the image 120 can be selected by moving the cursor over a point on image and clicking, for example with mouse 74 or 82. The selection of the point causes the point to be identified on the point navigation pane 112, as described in more detail below.

The point center button 138 can be operated to enter the point selection mode by pointing the cursor to the button and clicking, for example with mouse 74 or 82. In the point center mode the cursor might change in appearance, for example to a shape corresponding to the shape on the button 138. In this mode the view point for the image 120 can be centered upon a selected point by moving the cursor over a point on image and clicking, for example with mouse 74 or 82.

The autorotation button 140 can be operated to enter the autorotation mode by pointing the cursor to the button and clicking, for example with mouse 74 or 82. In the autorotation mode the cursor might change in appearance, for example to the shape corresponding to shape on the rotation button. In this mode the viewpoint for the image rotates automatically horizontally to the left. The direction of the rotation can be changed by pointing the cursor on the image and clicking and dragging in the desired new direction of rotation.

The image swap button 142 can be operated to swap the images displayed in the main pane 106 and in the subpane 124 by pointing the cursor to the button and clicking, for example with mouse 74 or 82.

The image capture button 144 can be operated to enter the image capture mode by pointing the cursor to the button, and clicking, for example with mouse 74 or 82. This opens a box that allows the user to save the image on the pane 106 for future reference.

The interface preferably displays a visual indicator of the desired orientation for the distal end of the medical device. In this first preferred embodiment, this indicator is an arrow 150, whose shaft is aligned with the desired orientation, with a large conical head pointing in the desired direction. The arrow 150 is preferably a distinctive color such as green. The interface preferably also displays a visual indicator of the current orientation of the distal end of the medical device. In this first preferred embodiment, this indicator is an arrow 152, whose shaft is aligned with the current orientation of the distal end of the medical device, with a larger conical head pointing in the desired direction.

A localization system could be provided for determining the current position and orientation of the distal end of the medical device. A image representative of the distal end of the medical device can then be generated and displayed in the pane 106. There are numerous method for localizing the distal end of the medical device, for example transmitting magnetic signals between the medical device and one or more reference locations, x-ray image processing, ultrasound localization, or electric potential localization.

In the first preferred embodiment, the interface is adapted for use with a magnetic navigation system that operates by generating a magnetic field of selected direction in the operating region, which causes a magnetically responsive element associated with the distal end of the medical device to generally align with the applied field. Because of the physical properties of the catheter, limitations in the strength of the applied field, and the conditions in the procedure site, the distal end of the medical device may not align precisely with the applied magnetic field. While the difference between the applied magnetic field and the actual direction of the distal end of the medical device can be accounted for through modeling or a look-up table, in the first preferred embodiment the arrow 150 representing the desired orientation may represent the desired direction of the applied magnetic field, rather than the desired direction of the medical device itself. Similarly, the arrow 152 representing the current orientation may represent the direction of the magnetic field to currently being applied, rather than the actual direction of the device itself. However, the differences between the actual direction of the medical device and the applied magnetic field can be characterized by equation or an empirically determined look-up table, or localization of the device can be provided so that even when used with a magnetic navigation system, the arrow 150 represents the actual desired orientation of the medical device, and arrow 152 represents the actual current direction.

To help visualize the three-dimensional direction of the indicator, the arrow 150 can be surrounded with an "umbrella" 154 ― a shape or surface surrounding the arrow so that its direction and orientation can be more easily visualized. One implementation of the umbrella 154 is as a wire frame hemisphere. In addition to improving the visualization of the direction of the arrow 150, the umbrella 154 can be used to selection the orientation of the arrow 150. When the cursor hovers over the surface of the umbrella, the cursor can change appearance, for example to resemble the rotation icon on button 134. The direction of the arrow 150 can be changed by rotating the hemisphere by pointing the cursor to the hemisphere, clicking, and dragging the cursor in the desired direction of rotation. In addition the arrow 150 and hemisphere 154 can be configured so that when the cursor hovers over the root of the arrow 150, the cursor can change in appearance, for example to resemble the translation icon on button 130. The position of the root of the arrow 150 can be changed by clicking the cursor and dragging the cursor in the desired direction of movement.

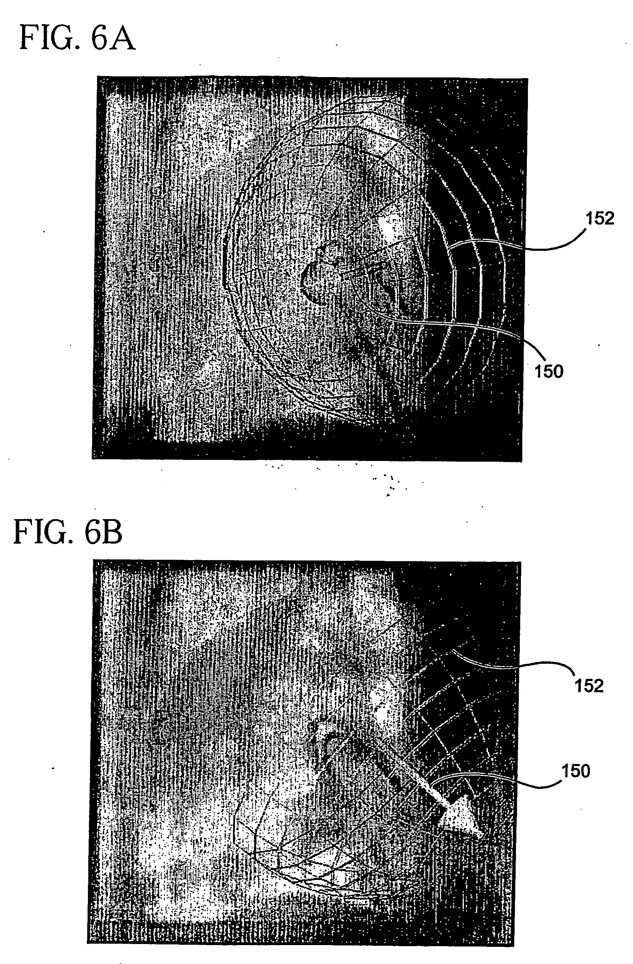

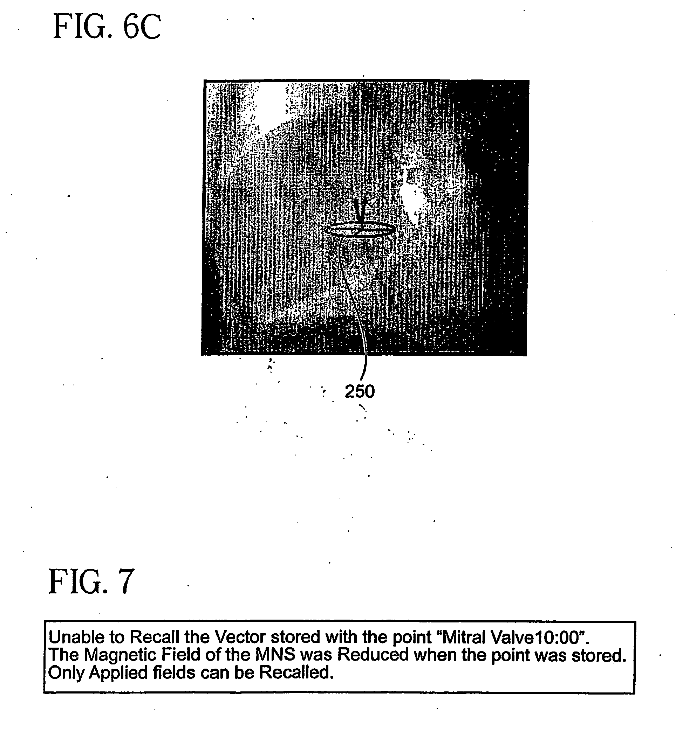

In the first preferred embodiment, the interface includes displays of the fluoroscopic images of the operating region, with the arrow 150 superposed thereon. For example, as shown in Figs. 6A and 6B, the imaging system 68 can provide biplanar images of the operating region, and the arrow 150 and umbrella 154 provided on each image. These images could be displayed on monitors 86 an 88 in the procedure room 50, and on monitor 90 in the control room 52. Preferably, the user can change the direction of the arrow 150 on these images as well by rotating and translating the arrow and umbrella as described above.

The display 100 of the interface preferably also includes a status area 108, where, as shown in Fig. 7, a text, graphic, or combination text and graphic message of the status of the interface can be displayed to the user. These messages can be colored coded for example to convey an immediate impression of the importance or significance of the message displayed.

While the orientation of the distal end of the medical device can be manipulated directly on the pane 106, for example by manipulating the umbrella 154, the display 100 of the interface preferably includes at least one pane to aid the user in selecting the desired orientation for the medical device. In this first preferred embodiment there are several panes provide alternative methods for the user to select the desired orientation for the distal end of the medical device. These panes include representations of the orientation of the arrow 150 which are constantly updated, so that use of one pane to change the desired direction of the medical device, causes all of the other panes to update, to facilitate the use of any of the panes to adjust the orientation of the arrow 150 representing the desired new orientation of the medical device.

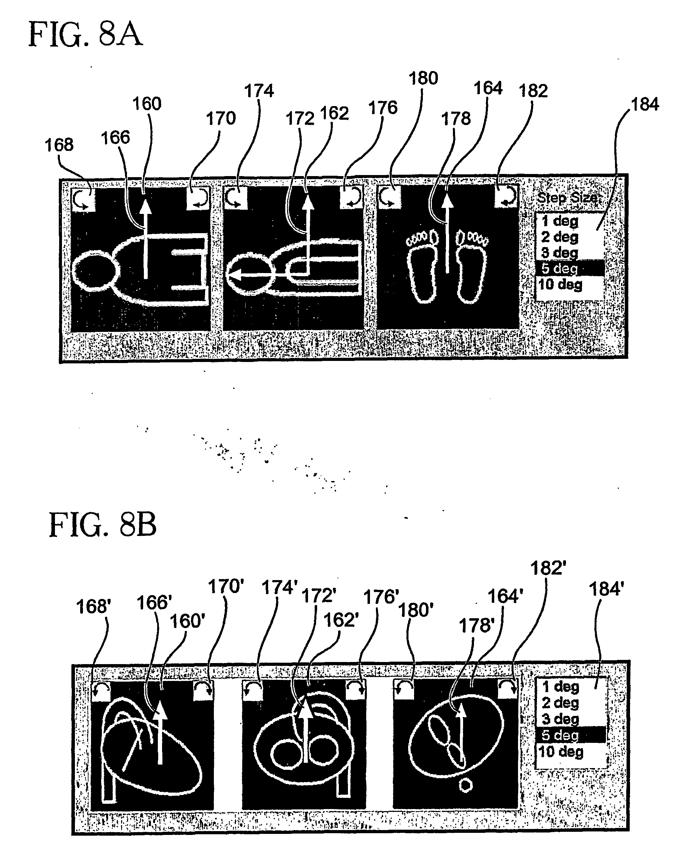

One such pane to aid the user in selecting the desired orientation for the medical device is the 2-D anatomical pane 110, which allows the user to select the desired orientation of distal end of the medical device as indicated by the arrow 150 by adjusting the direction in one or more planes through the patient. As shown in Fig. 8A, the pane 110 allows the user to change the direction of the arrow 150 in at least one plane, and preferably at least two planes and more preferably at least the planes. These planes are preferably, but not necessarily, mutually perpendicular. While adjustment in two planes is sufficient to specify any direction, providing adjustment in three planes makes it easier for a user to select the desired direction for the arrow 150. In this first preferred embodiment, the arrow can be rotated in the coronal or frontal plane (*i*.*e*., about an anterior-posterior axis), the median or saggital plane (*i.e.,* about a horizontal axis), and the horizontal or transverse plane (*i.e.,* about a longitudinal axis).

As shown in Fig. 8A the pane 110 can have three graphic displays 160, 162 and 164, corresponding to the three planes of rotation. Graphic display 160 contains a graphic depiction of the coronal or frontal plane (i.e., an caricature image of a patient's body in the coronal or frontal plane), with an indicator 166 that indicates the orientation of the arrow in the coronal or frontal plane, and virtual buttons 168 and 170 for moving the indicator 166 (and thus the arrow 150) clockwise or counterclockwise in the coronal or frontal plane abut the anterior-posterior axis. In this first preferred embodiment, indicator 166 is actually a projection of the arrow 150 in the plane, and thus the length of the indicator 166 is indicative of the orientation. The virtual buttons 168 and 170 can be operated with a cursor for example with the mouse 74 or 82 or the keyboard 80, to point and click the button and move the indicator 166 and thus the arrow 150, in the desired direction. Display 162 contains a graphic depiction of the median or saggital plane (i.e., a caricature image of a patient's body in the median or saggital plane), with an indicator 172 indicating the direction of the arrow 150 in the median or saggital plane, and virtual buttons 174 and 176 for moving the indicator 172 (and thus the arrow 150) clockwise or counterclockwise in the coronal or frontal plane. In this first preferred embodiment, indicator 172 is actually a projection of the arrow 150 in the plane, and thus the length of the indicator 172 is indicative of the orientation. The virtual buttons 174 and 176 can be operated with a cursor for example with the mouse 74 or 82 or the keyboard 80, to point and click and move the indicator 172 and thus the arrow 150, in the desired direction. Display 164 contains a graphic depiction of the horizontal or transverse plane (i.e., a caricature image of a patient's body in the horizontal or transverse plane), with an indicator 178 indicating the direction of the arrow in the horizontal or transverse plane, and virtual buttons 180 and 182 for moving the indicator 178 (and thus the arrow 150) clockwise or counterclockwise in the horizontal or transverse plane. The virtual buttons 180 and 182 can be operated with a cursor for example with the mouse 58 or 66 or the keyboard 64, to point and click to move the indicator 178, and thus the arrow 150 in the desired direction.

The pane 110 also includes a menu 184 to select the increment of change in direction upon operating the buttons 168 and 170, 174 and 176, and 180 and 182. The user can select the incremental change from 1 degree, 2 degrees, 3 degrees, 5 degrees, 10 degrees with a cursor for example with the mouse 74 or 82 or the keyboard 80, to point and click to select the desired increment.

An alternate implementation of the pane 110' is shown Fig. 8B. In contrast to Fig. 8A where pane 110 allows movement of the arrow 150 relative to the coronal or frontal plane, the median or saggital plane, and the horizontal or transverse plane, in Fig. 8B the pane 110' allows movement of the arrow 150 relative to the right anterior oblique plane, the left anterior oblique plane, and the transverse plane. As shown in Fig. 8B the pane 110 can have three graphic displays 160', 162' and 164', corresponding to the three planes of rotation. Graphic display 160' contains a graphic depiction of the right anterior oblique plane (i.e., an caricature image of patient's body or part of the patient's body in the RAO plane), with an indicator 166' that indicates the orientation of the arrow in the coronal or frontal plane, and virtual buttons 168' and 170' for moving the indicator 166' (and thus the arrow 150) clockwise or counterclockwise in the left anterior oblique plane. In this first preferred embodiment, indicator 166' is actually a projection of the arrow 150 in the plane, and thus the length of the indicator 166' is indicative of the orientation. The virtual buttons 168' and 170' can be operated with a cursor for example with the mouse 74 or 82 or the keyboard 80, to point and click the button and move the indicator 166' and thus the arrow 150, in the desired direction. Display 172' contains a graphic depiction of the left anterior oblique plane (i.e., a caricature image of a patient's body or portion of the patient's body in the LAO plane), with an indicator 172' indicating the direction of the arrow 150 in the median or saggital plane, and virtual buttons 174' and 176' for moving the indicator 172' (and thus the arrow 150) clockwise or counterclockwise in the coronal or frontal plane. In this first preferred embodiment, indicator 172' is actually a projection of the arrow 150 in the plane, and thus the length of the indicator 172' is indicative of the orientation. The virtual buttons 174' and 176' can be operated with a cursor for example with the mouse 74 or 82 or the keyboard 80, to point and click and move the indicator 172' and thus the arrow 150, in the desired direction. Display 164' contains a graphic depiction of the horizontal or transverse plane (*i*.*e*., a caricature image of a patient's body or a portion of the patient's body in the horizontal or transverse plane), with an indicator 178' indicating the direction of the arrow in the horizontal or transverse plane, and virtual buttons 180' and 182' for moving the indicator 178' (and thus the arrow 150) clockwise or counterclockwise in the horizontal or transverse plane. The virtual buttons 180' and 182' can be operated with a cursor for example with the mouse 58 or 66 or the keyboard 64, to point and click to move the indicator 178', and thus the arrow 150 in the desired direction.

The pane 110' also includes a menu 184' to select the increment of change in direction upon operating the buttons 168' and 170', 174' and 176', and 180' and 182'. The user can select the incremental change from 1 degree, 2 degrees, 3 degrees, 5 degrees, 10 degrees with a cursor for example with the mouse 74 or 82 or the keyboard 80, to point and click to select the desired increment.

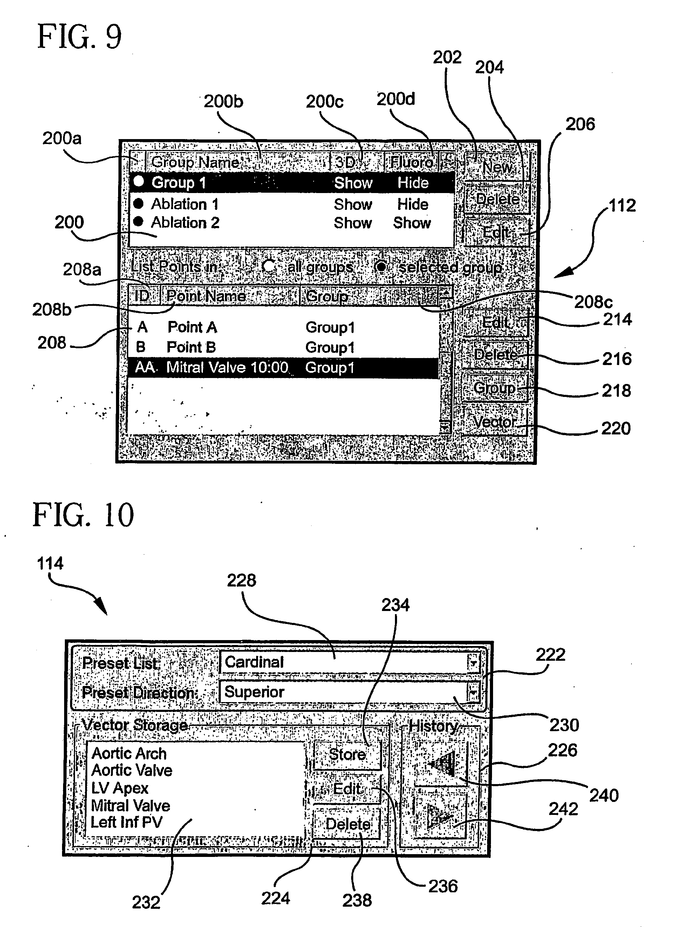

Another pane to aid the user in selecting the desired orientation for the arrow 150 and thus for the medical device is a point navigation pane 112. As shown in Fig. 9, the point navigation pane 112 includes a group menu table 200 containing information about one or more groups of points the user identifies. The group menu table 200 includes a column 200a with a color indicator for indicating the color corresponding to the points in the group. All points in the group will be indicated with a mark in the indicated color. The menu table 200 further includes a column 200b entitled "Group Name" with the name of a stored group of points. The menu table 200 further includes a column 200c entitled "3D" which indicates whether the group of points is visible on the 3D display in pane 106 ("show") or not visible on the 3D display in the pane ("hide"). Finally, the table comprises a column 200 entitled "Fluro" which indicates whether the group of points is visible on the 3D display in pane 106 ("show") or not visible on the 3D display in the pane ("hide").

A "new" button 202, a "delete" button 204, and an "edit" button 206 are associated with the menu table 200. The buttons 202, 204, and 206 are preferably "virtual" buttons, i.e. portions of the display on which the user points the cursor and clicks, for example with mouse 74 or 82, or keyboard 82. The new button 202 can be operated by pointing and clicking with the cursor using the mouse 74 or 82 or keyboard 80, and allows the user to create a new group in the menu table 200. Operating the new button 202 opens a box that allows the user to select the color indicator in column 200a, select the name of the group in column 200b, select the display properties in column 200c between "show" and "hide" to determine whether the points will appear on the 3D panel 110, and select the display properties in column 200d, between "show" and "hide" to determine whether the points will appear on the fluoroscope displays (monitors 86, 88, and 90). The delete button 204 can be operated by pointing and clicking with the cursor using the 74 or 82, or keyboard 82, and allows the user to delete the group or groups that the user highlighted in the menu table 114, using the mouse 74 or 82, or keyboard 82. The edit button 206 can be operated by pointing and clicking with the cursor using the mouse 74 or 82, or keyboard 82, and allows the user to edit the group that the user highlighted in the menu table 200 using the 74 or 82, or keyboard 82. Operating the edit button 206 opens a box that allows the user to change the color indicator in column 200a, change the name of the group in column 200b, change the display properties in column 200c between "show" and "hide" to determine whether the points will appear on the 3D panel 110, and change the display properties in column 200d, between "show" and "hide" to determine whether the points will appear on the fluoroscope displays (monitors 86, 88, and 90).

The pane 112 also includes a point menu table 208. The menu table 208 includes a column 208a, entitled "id" for an identification code assigned by the system to a particular point (in the first preferred embodiment the system assigns an id from A to ZZ). The menu table 208 further includes a column 208b, entitled "point name" for the name of the point. Finally, the menu table 208 includes a third column 208c entitled "group" for the name of the group to which the point is assigned. A display control is provided adjacent the point menu table 208 for selection the points to display in the point menu table 208. As shown in Fig. 9, the display control can comprise radio buttons 210 and 212, which allow the user to specify "all groups" or "selected group", respectively, so that the user can identify whether to display the points in "all groups" or just the points a selected group "selected group" in the menu table 208.

An "edit" button 214, a "delete" button 216, a "group" button 218, and a "vector" button 220 are associated with the menu table 208. The buttons 214, 216, 218, and 220 are preferably "virtual" buttons on the display that can be operated by pointing the cursor and clicking, for example with mouse 74 or 82, or keyboard 80. The user can select a point on the menu table 200 by pointing with the cursor and clicking, using the muse 74 or 82, or the keyboard 80. The edit button 214 can be operated by pointing and clicking with the cursor using the mouse 74 or 82 or keyboard 80, and allows the user to edit the selected point. Operating the edit box opens a box that allows the user to change the name of the selected point in column 208b, and the group to which the point is column 208c. The delete button 216 can be operated by pointing and clicking with the cursor using the mouse 74 or 82 or keyboard 80, and allows the user to delete the selected point. The group button 218 can be operated by pointing and clicking with the cursor using the mouse 74 or 82 or keyboard 80, and allows the user to change the group to which the selected point is associated. The vector button 220 can be operated by pointing and clicking with the cursor using the mouse 74 or 82 or keyboard 80, and allows the user to set the orientation of the arrow 150 to the orientation associated with a point selected on the menu table 208 using the mouse 74 or 82 or keyboard 80. This automatically updates the display of arrow 150 in the other panes. Thus a user who wants to navigate back to a stored point can recall the direction associated with that point, facilitating the return to the point. However that direction may also be useful in navigating to another point.

Another such pane to aid the user in selecting the desired orientation for the medical device is a vector navigation pane 114. The vector navigation pane 114 allows the user to use predetermined directions, to store and use new directions, and to recall and use previously used directions. The vector navigation pane 114 includes a section 222 for recalling and using predetermined directions; a direction vector storage and recall section 224; and a direction history and recall section 226. The section 222 for recalling and using predetermined directions includes a "preset list" pick menu 228 for selecting a particular set of predetermined directions, and a "direction" pick menu 230 for selecting a particular direction from the selected set. A set of possible "preset list" and "direction" entries for the pick menus 228 and 230 is shown in Table 1. The user can select from the "preset list" and "direction" pick menus using the mouse 74 or 82 or keyboard 80.

| Table 1― Possible Preset Lists and Directions | |
|---|---|
| Cardinal | Superior |
| Cardinal | Inferior |
| Cardinal | Anterior |
| Cardinal | Posterior |
| Cardinal | Left |
| Cardinal | Right |
| Cardinal | RAO |
| Cardinal | LAO |
| Deflection | Right from 0 to 330 n 15° increments |

The direction vector storage and recall section 224 includes a vector menu table 232, and associated "store" button 234, "edit" button 236, "delete" button 238. The buttons 234, 236, and 238 are preferably virtual buttons, or portions of the display to which the cursor can be pointed and clicked, for example with the mouse 74 or 82, or the keyboard 80. The "store" button 234 can be operated by pointing and clicking with the cursor using the mouse 74 or 82 or keyboard 80, and allows the user to store the current direction under a user selected name on the vector menu table 232. Operating the store button 234 opens a box that allows the user to input a name. The user can selected a stored direction from the menu table 232 by pointing to the name with the cursor, and clicking, using the mouse 74 or 82, or keyboard 80. The "edit" button 236 can be operated by pointing and clicking with the cursor using the mouse 74 or 82 or keyboard 80, and allows the user to edit the name of a selected direction. The "delete" button 238 can be operated by pointing and clicking with the cursor using the mouse 74 or 82 or keyboard 80, and allows the user to delete a selected direction. The history section 226 includes virtual forward and back buttons 240 and 242. The forward and back buttons 240 and 242 can be operated by pointing the cursor and clicking using mouse 74 or 82 or keyboard 80. The buttons 240 and 242 allow the user to set the orientation of the arrow 150 to one of the previously selected directions, which are automatically stored. In the first preferred embodiment, the system automatically stores the last ten directions, and the user can scroll backward and forward through these directions with the buttons 240 and 242. The appearance of the buttons 240 and 242 changes (e.g. grays out) when the there is no further stored directions.

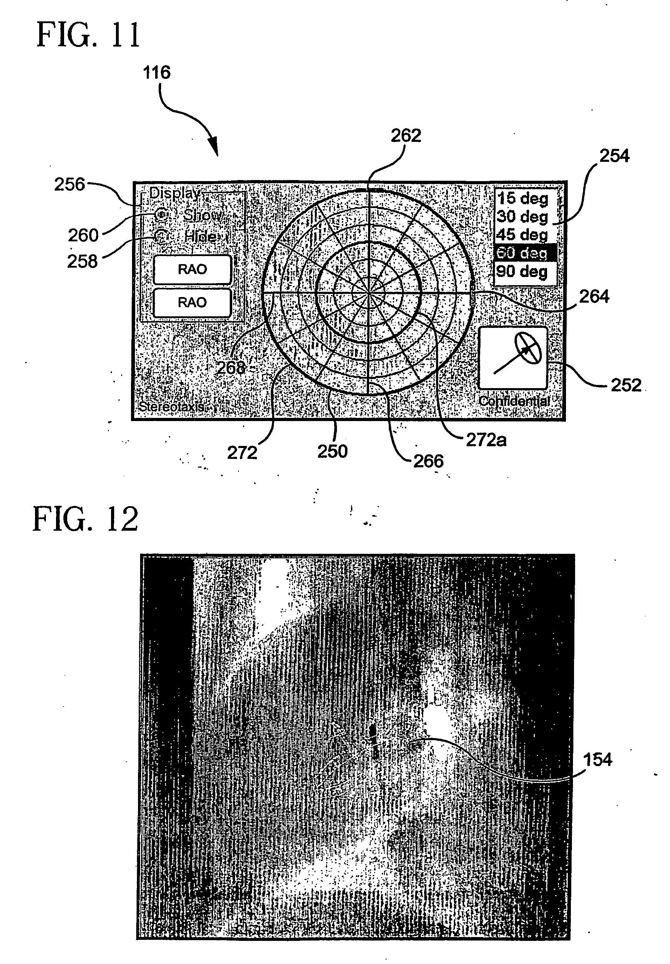

The bull's eye navigation pane 116 includes a circular screen 250, and an "apply" button 252. The pane 116 also includes a scale menu 254, which in the first preferred embodiment allows the user to select the scale of the screen 250 from 15, 20, 45, 60, and 90 degrees. The user can select the desired scale for the circular screen 250 by pointing the cursor and clicking, using the mouse 74 or 82 or keyboard 80. The pane 116 may also include a display control section 256 with "Hide" and "Show" radio buttons 258 and 260. These buttons determine whether the circular screen 250 is projected onto the other displays, specifically the 3D display of pane 106 and the fluoroscopic images from the imaging system displayed on the monitors 86, 88, and 90. Fig. 12 shows one of the biplane imaging displays with the screen 250 projected thereon. The display control section 256 also includes RAO (right anterior oblique) and LAO (left anterior oblique) selection buttons 262 and 264, which orient the screen 250 so that the top of the screen is up in whichever of the two views is selected. As shown in Fig. 11, markers 262 and 264 are provided on the circular screen 250, to help the user interpret the orientation of the circular screen 250 on the 3D pane 106 and the on the RAO and LAO views. The marker 262 might be blue and the marker 264 might be red.

The user can set the base direction the navigation pane 116 by operating the "apply" button 252 by pointing at the button with a cursor and clicking, using mouse 74 or 82 or keyboard 80. The sets the current direction as the direction though the center of the screen 250. The user can then specify a direction for the arrow 150 by selecting a point on the screen 250, by pointing with the cursor and clicking, using mouse 74 or 82, or keyboard 80. As shown in Fig. 11, the screen 250 has vertical and horizontal cross hairs 266 and 268, and a plurality of radially extending markers 270, at 30 degree intervals. There are a plurality of concentric circular markers 272 representing regular angular intervals (10 degree intervals in the first preferred embodiment), with specified intervals (30 degree intervals in the first preferred embodiment) indicated by bold markers 272a. The circular screen 272 actually represents a hemisphere of space. The screen allows the user to orient the arrow 150 at a number of points to draw radial and circular lines.

The toolbar 104 preferably also includes an indicator 280, an apply button 282, a reduce button 284, and an angle indicator 286. The indicator 280 indicates when the interface is connected to the magnetic navigation system. Of course if some other system for orienting the distal end of the medical device is used, a suitable indicator can be provided. The apply button 282 and the reduce button 284 are preferably virtual buttons which are operated by pointing the cursor and clicking, for example with mouse 74 or 82, or keyboard 80. Operating the apply button 282 causes the magnetic navigation system to apply a magnetic field to orient the distal end of the medical device in the orientation of the arrow 150. Operating the reduce button 284 causes the magnetic navigation system to "turn off' the magnetic field. The indicator 286 indicates the angular difference between the previously applied magnetic field and the orientation of arrow 150. Of course rather than discrete navigation, in which the arrow 150 is successively oriented and the magnetic field applied, the interface could be adapted to operate in a continuous navigation mode in which the field is automatically applied in the direction of arrow 150

### Operation

In operation the user can visualize the current direction of the device represented by arrow 154 and the desired new direction for the device represented by arrow 150, on the 3-D pane 106 or on the x-ray images on monitors 86. 88, and 90. The user can selected the orientation of the arrow 150 in a number of ways using panes 110, or 112, or 114, or 116.

The user can select the orientation of arrow 150 on pane 110 by clicking on buttons 168 and 170, 174 and 176, and 180 and 182, to move the arrow 150 in each of the coronal or frontal plane, the median or saggital plane, and the horizontal or transverse plane to move the arrow. Alternatively, the user can select the orientation of arrow 150 by using the pane 112. The user selects a point on the menu table 208 by pointing and clicking with the cursor, and then operating the vector button, 220 by pointing and clicking with the cursor. This sets the orientation of arrow 150 to the orientation associated with point selected. Alternatively, the user can select the orientation of arrow 150 using the pane 114. The user can select a stored orientation by selecting a category on menu 228, and a direction on menu 230. The user can select a user-stored direction by selecting a direction vector from the menu table 232. The user can select a previously used direction by using the buttons 240 and 242 to recall one of the last previously used direction. Finally, the user can select an orientation by picking a point on a screen 250.

Once the direction of the arrow 150 is selected, the navigation system can be operated by operating the apply button 282. This can operate a magnetic navigation system to apply a field in the direction 150, or it can operate a magnetic navigation system to apply a field to cause the medical device to align in the direction 150, either by using feedback of the catheter position or by calculating or using a look-up table to account for the properties of medical device.

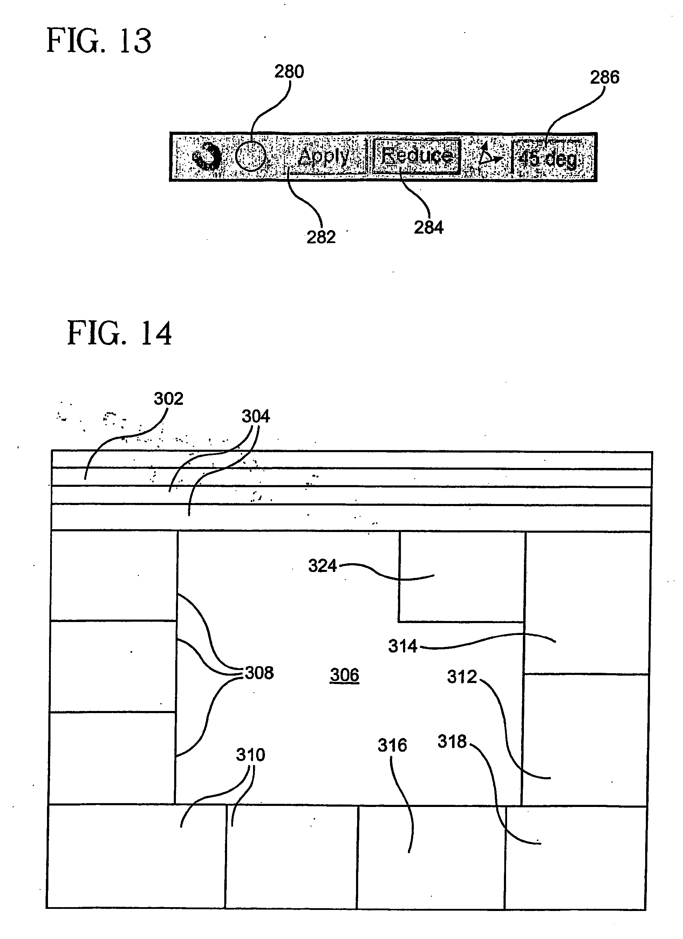

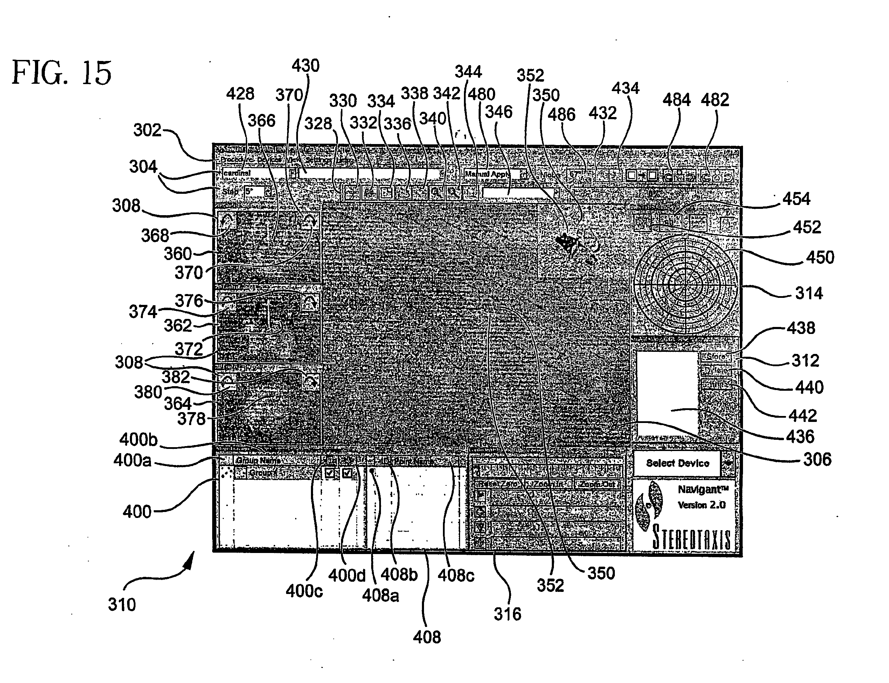

In second preferred embodiment, as shown in Figs. 14 the display 100' on the monitors 72 and 78, includes a menu bar 302, tool bars 304, a 3-D display pane 306, a 2-D anatomical control pane 308, a point navigation control pane 310, a vector navigation control pane 312, and a bull's eye navigation control pane 314, an advancer control pane 316, and a title block and device selection pane 318. Of course the display 100' could include additional panes or fewer panes or different panes. An example of a display in accordance with this invention is shown in Fig. 15.

A 3-D display pane 306 in accordance with this invention is shown in Fig. 15. The display preferably includes a three-dimensional representation of the patient orientation. As shown in Fig. 15 this representation may be a representation of a horizontal grid corresponding to the surface of the patient support 56. Alternatively, the may be a three dimensional representation of an idealized patient, or of the patient support 56. The pane 306 preferably also includes a subpane 324 that displays three dimensional representation of the operating region. In this preferred embodiment this representation is an transparent, three dimensional idealized representation of the portion of the patient's body in which the procedure is taking place, e.g. a human heart as shown in Fig. 15. To facilitate the user's interpretation of the image, the image may be displayed over a horizontal backing grid. Instead of an idealized representation of the procedure site, the image could be an actual preoperative image, or a actual current image. A coordinate system is optionally included in the representation to facilitate the user's understanding of the orientation.

The tool bar 304 includes a 3D tool bar 328 with controls for controlling the 3-D display pane 306. In this second preferred embodiment, these controls include a screen manipulation button 330, a grid button 332, a display selector button 334, a constellation button 336; a point centering button 338, a zoom in button 340, a zoom out button 342, and an image capture button 344. These buttons are preferably "virtual buttons" , *i*.*e*., they are elements on the display which the user can operate by pointing a cursor and clicking.

A view selection menu bar 346 is also provided on the 3D tool bar 328. The view selection menu 346 has an arrow that can be operated to drop down a menu of views to display in the pane 306. These preferably include cranial, caudal, anterior, posterior, left and right, as well as one or more user defined views. Of course other standard views could be provided depending upon the procedures for which the interface is used.

The screen manipulation button 330 can be actuated (for example by right clicking) to display a plurality of screen manipulation options for the cursor. For example, the user can select among a plurality of cursor modes to translate the image on the display 306, to rotate the image on the display, etc., by clicking and dragging the image. The appearance of the cursor on the display 306 preferably changes to cue the user as to the particularly screen manipulation mode in effect. In the translation mode, the cursor might change in appearance, for example to a shape corresponding to the icon on the button 330. In this mode the view point can be changed by grabbing the image by clicking when the cursor is on the image, and dragging the cursor to move the image and thus the viewpoint in any direction. The cursor can be moved using mouse 74 or 82. This preferably also causes a corresponding translation of the view point of the image in the subpane 324.

The grid button 332 can be clicked to show and hide the grid lines on the display 306.

The display selector button 334 allows the user to select the format of the display 306. The user can click on the button to cause a menu of icons depicting various formats to drop down. The user then simply selects the desired format, for example including the subpane 324 (as shown) or removing the subpane 324.

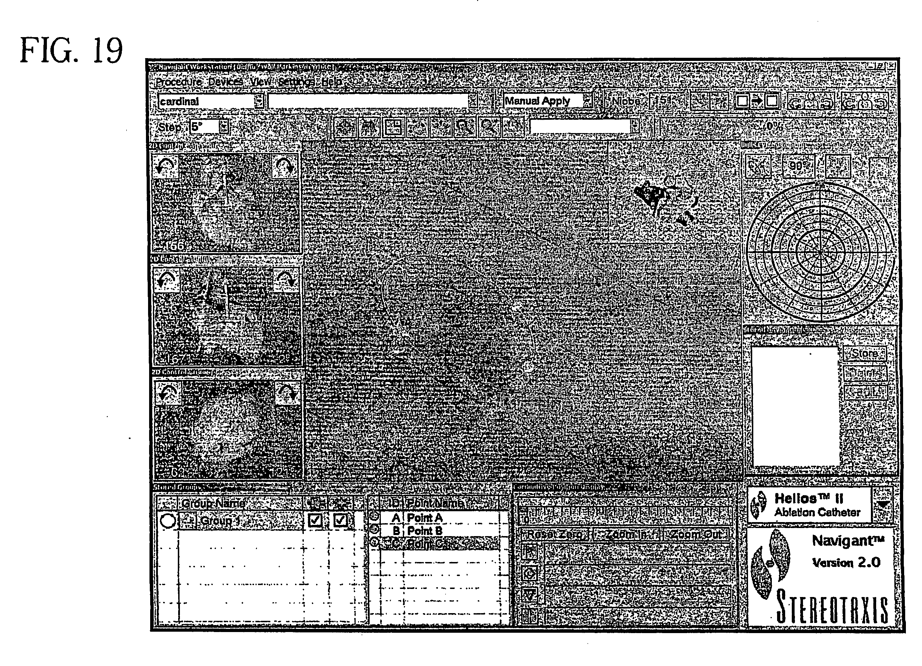

The display constellations button 336 can be operated to toggle between a display in which points on the display 306 are shown as part of a group or constellation (e.g. Fig. 19) by pointing the cursor to the button and clicking, for example with mouse 74 or 82.

The point center button 338 can be operated to enter the point selection mode by pointing the cursor to the button and clicking, for example with mouse 74 or 82. In the point center mode the cursor might change in appearance, for example to a shape corresponding to the shape on the button 338. In this mode the view point for the image can be centered upon a selected point by moving the cursor over a point on image and clicking, for example with mouse 74 or 82.

The zoom in button 340 allows the user to click to enlarge the image on the display 306, and the zoom out button 342 allows the user to click to reduce the image on the display 306 The zoom in button 340 and the zoom out button 342 can be operated to enter the magnification or zoom mode by pointing the cursor to the button and clicking for example with mouse 74 or 82. In the zoom mode the cursor might change in appearance, for example to a shape corresponding to the magnifying glass icon with a "+" for zoom in, and a "-" for zoon out. In this mode the magnification of the image can be accomplished by grabbing the image by pointing the cursor and clicking, and dragging the cursor downwardly and/or to the right to increase the magnification, or upwardly or to the left to decrease the magnification. Changing the size of the patient reference image 306 preferably also does not change the size of the procedure site reference image. 324

The image capture button 344 can be operated to enter the image capture mode by pointing the cursor to the button, and clicking, for example with mouse 74 or 82. This opens a box that allows the user to save the image on the pane 306 for future reference.

The interface preferably displays a visual indicator of the desired orientation for the distal end of the medical device. In this preferred embodiment, this indicator is an arrow 350, whose shaft is aligned with the desired orientation, with a large conical head pointing in the desired direction. The arrow 350 is preferably a distinctive color, e.g. green. The interface preferably also displays a visual indicator of the current orientation of the distal end of the medical device. In this preferred embodiment, this indicator is an arrow 352, whose shaft is aligned with the current orientation of the distal end of the medical device, with a larger conical head pointing in the desired direction. The arrow 352 is preferably a distinctive color, different from the arrow 350, e.g. yellow.

A localization system could be provided for determining the current position and orientation of the distal end of the medical device. An image representative of the distal end of the medical device can then be generated and displayed in the pane 306. There are numerous method for localizing the distal end of the medical device, for example transmitting magnetic signals between the medical device and one or more reference locations, x-ray image processing, ultrasound localization, or electric potential localization.

In the preferred embodiment, the interface is adapted for use with a magnetic navigation system that operates by generating a magnetic field of selected direction in the operating region, which causes a magnetically responsive element associated with the distal end of the medical device to generally align with the applied field. Because of the physical properties of the catheter, limitations in the strength of the applied field, and the conditions in the procedure site, the distal end of the medical device may not align precisely with the applied magnetic field. While the difference between the applied magnetic field and the actual direction of the distal end of the medical device can be accounted for through modeling or a look-up table, in the preferred embodiment the arrow 350 representing the desired orientation may represent the desired direction of the applied magnetic field, rather than the desired direction of the medical device itself. Similarly, the arrow 352 representing the current orientation may represent the direction of the magnetic field to currently being applied, rather than the actual direction of the device itself. However, the differences between the actual direction of the medical device and the applied magnetic field can be characterized by equation or an empirically determined look-up table, or localization of the device can be provided so that even when used with a magnetic navigation system, the arrow 350 represents the actual desired orientation of the medical device, and arrow 352 represents the actual current direction.

As in the first preferred embodiment, in the second preferred embodiment, the interface includes displays of the fluoroscopic images of the operating region, with the arrow 350 superposed thereon. For example, as shown in Figs. 6A and 6B, the imaging system 68 can provide biplanar images of the operating region, and the arrow 350 on each image. These images could be displayed on monitors 86 an 88 in the procedure room 50, and on monitor 90 in the control room 52. Preferably, the user can change the direction of the arrow 150 on these images by rotating and translating the arrow as described above.

While the orientation of the distal end of the medical device can be manipulated directly on the pane 306, the display 100' of the interface preferably includes at least one pane to aid the user in selecting the desired orientation for the medical device, and thus of the arrow 350. In this preferred embodiment there are several panes that provide alternative methods for the user to select the desired orientation for the distal end of the medical device. These panes include representations of the orientation of the arrow 350 which are constantly updated, so that use of one pane to change the desired direction of the medical device, causes all of the other panes to update, to facilitate the use of any of the panes to adjust the orientation of the arrow 350 representing the desired new orientation of the medical device.

One such pane to aid the user in selecting the desired orientation for the medical device is the 2-D anatomical pane 308, which allows the user to select the desired orientation of distal end of the medical device as indicated by the arrow 350 by adjusting the direction in one or more planes through the patient. As shown in Fig. 15, the pane 310 allows the user to change the direction of the arrow 350 in at least one plane, and preferably at least two planes and more preferably at least the planes. These planes are preferably, but not necessarily, mutually perpendicular. While adjustment in two planes is sufficient to specify any direction, providing adjustment in three planes makes it easier for a user to select the desired direction for the arrow 350. In this preferred embodiment, the arrow 350 can be rotated in the coronal or frontal plane (i.e., about an anterior-posterior axis), the median or saggital plane (i.e., about a horizontal axis), and the horizontal or transverse plane (i.e., about a longitudinal axis).

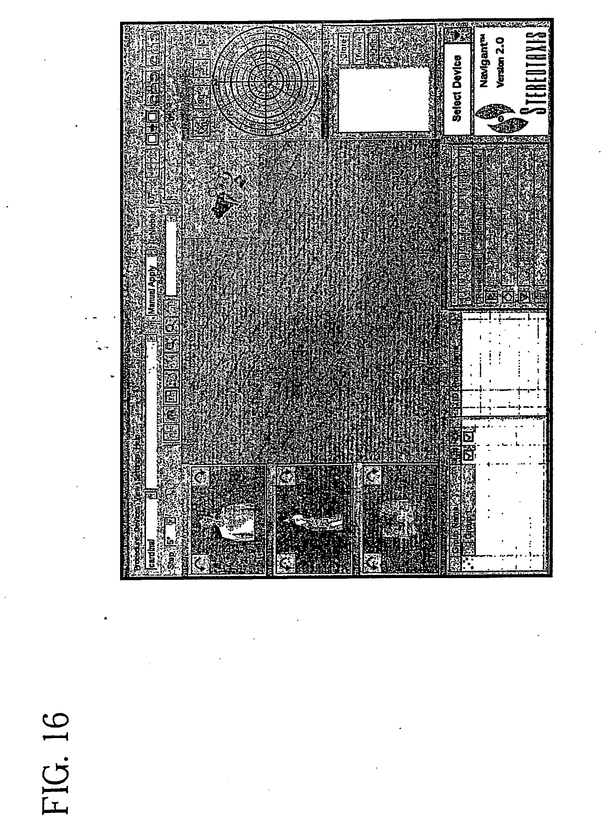

As shown in Figs. 15-19 the pane 308 can have three graphic displays 360, 362 and 364, corresponding to the three planes of rotation. As shown in Figs. 15 and 16, the user can preferably select between an anatomy view (Fig. 15) or a whole body view (Fig. 16). Graphic display 360 contains a graphic depiction of the coronal or frontal plane (e.g. a caricature image of the organ and/or operation region or a caricature image of a patient's body, in the coronal or frontal plane), with an indicator 366 that indicates the orientation of the arrow in the coronal or frontal plane, and virtual buttons 368 and 370 for moving the indicator 366 (and thus the arrow 350) clockwise or counterclockwise in the coronal or frontal plane abut the anterior-posterior axis. In this preferred embodiment, indicator 366 is actually a projection of the arrow 350 in the plane, and thus the length of the indicator 366 is indicative of the orientation. The virtual buttons 368 and 370 can be operated with a cursor for example with the mouse 74 or 82 or the keyboard 80, to point and click the button and move the indicator 366 and thus the arrow 350, in the desired direction. Display 362 contains a graphic depiction of the median or saggital plane (i.e., a caricature image of a patient's body in the median or saggital plane), with an indicator 372 indicating the direction of the arrow 350 in the median or saggital plane, and virtual buttons 374 and 376 for moving the indicator 372 (and thus the arrow 350) clockwise or counterclockwise in the coronal or frontal plane. In this preferred embodiment, indicator 372 is actually a projection of the arrow 350 in the plane, and thus the length of the indicator 372 is indicative of the orientation. The virtual buttons 374 and 376 can be operated with a cursor for example with the mouse 74 or 82 or the keyboard 80, to point and click and move the indicator 372 and thus the arrow 350, in the desired direction. Display 364 contains a graphic depiction of the horizontal or transverse plane (i.e., a caricature image of a patient's body in the horizontal or transverse plane), with an indicator 378 indicating the direction of the arrow in the horizontal or transverse plane, and virtual buttons 380 and 382 for moving the indicator 378 (and thus the arrow 350) clockwise or counterclockwise in the horizontal or transverse plane. The virtual buttons 380 and 382 can be operated with a cursor for example with the mouse 58 or 66 or the keyboard 64, to point and click to move the indicator 278, and thus the arrow 250 in the desired direction.

The pane 308 also includes a menu 384 to select the increment of change in direction upon operating the buttons 368 and 370, 374 and 376, and 380 and 382. The user can select the incremental change from 1 degree, 2 degrees, 3 degrees, 5 degrees, 10 degrees with a cursor for example with the mouse 74 or 82 or the keyboard 80, to point and click to select the desired increment.

Instead of using controls 368 and 370, 374 and 376, and 380 and 382, to incrementally move the indicators 366, 372. and 378, the user can simply point and click on the three graphic displays 360, 362 and 364 to move the indicator to the selected point. Moving the indicators either with controls 368 and 370, 374 and 376, and 380 and 382, or by selecting points on the displays 366, 372, and 378, the user can selected the direction of arrow 350.

Another pane to aid the user in selecting the desired orientation for the arrow 350, and thus for the medical device, is point navigation pane 310. As shown in Fig. 15, the point navigation pane 310 includes a group menu table 400 containing information about one or more groups of points the user identifies. The group menu table 400 includes a column 400a for an icon for identifying the arrangement of the group (a group of points can be thought of as defining a shape, such as a circle, ellipse, or spline much the same way that stars for constellations of shapes). An icon representing unorganized points is shown in Fig. 15, a icon representing an ellipse "constellation" is shown in Fig. 19. Other types of arrangements of points in a group, for example points on fitted curve, and points on a spline, can be identified with different icons in the column 400a. A column 400b, with the heading "Group Name" includes a color/shape identifier and a name for the group, e.g. "Group 1". In this second preferred embodiment, a square of a color identifying the group is displayed in column 400b, but the group could be identified in some other manner. All points in a group will be indicated with a mark in the indicated color, as described in more detail below. The group menu table 400 further includes a column 400c for a pick box for each group for indicating whether the group should be shown on the bi-plane fluoroscopic imaging screens (on monitors 86, 88, and 90), and a column 400d for a pick box for each group for indicating whether the group should be shown on the 3D display in pane 306.

The identified of points, groups of points, and constellations of points within a group allows the user to simply identify a point or points and have the interface determine the field direction to reach the point or points

The pane 310 also includes a point menu table 408. The menu table 408 includes a column 408a, for an identification symbol that indicates (preferably using color) the group to which the point belongs, a column 408b entitled "ID" that contains a code assigned by the system to a particular point (in this second preferred embodiment the system assigns an ID sequentially from A to ZZ). The menu table 208 further includes a column 208c, entitled "Point Name" for a user specified name of the point. The user can select a group by pointing the cursor on a group in the group menu table 400, which causes the point menu table 408 to display each of the points in the selected group.

As a further aid to the user in selecting the desired orientation for the medical device, vector navigation pick menus 428 and 430 are provided on the toolbars 304. The pick menu 428 displays a "preset list" pick menu for selecting a particular set of predetermined directions, and the pick menu 430 displays a "direction" pick menu for selecting a particular direction from the set selected in window 428. A set of possible "preset list" and "direction" entries for the pick menus 428 and 430 is shown in Table 2. The user can select from the "preset list" and "direction" pick menus using the mouse 74 or 82 or keyboard 80.

| Table 2 ― Possible Preset Lists and Directions | |
|---|---|
| Cardinal | Superior |
| Cardinal | Inferior |
| Cardinal | Anterior |
| Cardinal | Posterior |
| Cardinal | Left |
| Cardinal | Right |
| Cardinal | RAO |
| Cardinal | LAO |
| Deflection | Right from 0 to 330 n 15° increments |

Vector history buttons 432 and 434 are also provided on one of the tool bars 304 to aid the user in selecting the desired orientation for the medical device. The buttons 432 and 434 allow the user to move backwardly and forwardly through an automatically stored list of applied magnetic field directions, in order to reapply a previously applied magnetic field. The buttons 432 and 434 allow the user to set the orientation of the arrow 350 to one of the previously selected directions, which are automatically stored In the preferred embodiment, the system automatically stores the last ten directions, and the user can scroll backward and forward through these directions with the buttons 432 and 434. The appearance of the buttons 432 and 434 preferably changes (e.g. grays out) when the there is no further stored directions.

The interface can also include a vector storage and recall pane 312 to store, recall, and use custom directions. The direction vector storage and recall pane 312 includes a vector menu table 436, and associated "store" button 438, "delete: button 440, and "edit" button 442. The buttons 438, 440, and 442 are preferably virtual buttons, or portions of the display to which the cursor can be pointed and clicked, for example with the mouse 74 or 82, or the keyboard 80. The "store" button 438 can be operated by pointing and clicking with the cursor using the mouse 74 or 82 or keyboard 80, and allows the user to store the current direction under a user selected name on the vector menu table 436. Operating the store button 438 allows the user to input a name for the stored direction. The user can selected a previously stored direction from the menu table 436 by pointing to the name with the cursor, and clicking, using the mouse 74 or 82, or keyboard 80. The "edit" button 442 can be operated by pointing and clicking with the cursor using the mouse 74 or 82 or keyboard 80, and allows the user to edit the name of a selected direction. The "delete" button 440 can be operated by pointing and clicking with the cursor using the mouse 74 or 82 or keyboard 80, and allows the user to delete a selected direction.

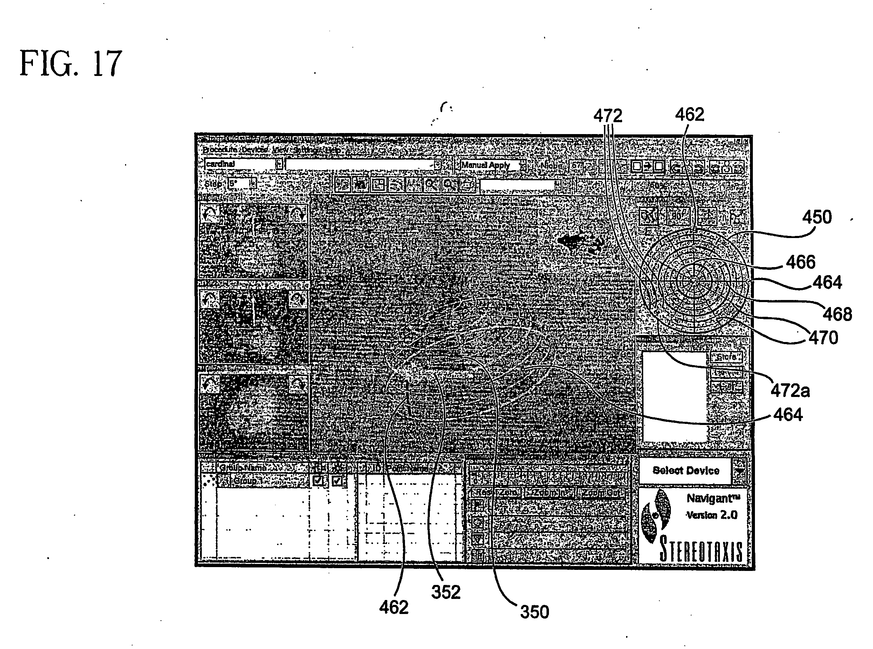

The bull's eye navigation pane 314 includes a circular screen 450, and an "apply" button 452. The pane 314 also includes a scale menu 454, which in the preferred embodiment allows the user to select the scale of the screen 450 from 15, 20, 45, 60, 90, and 120 degrees. The user can select the desired scale for the circular screen 250 by pointing the cursor at the scale menu 454, to display a list of scales, and selecting and clicking on the desired scale, using the mouse 74 or 82 or keyboard 80. Fig. 17 shows the circular screen 450 on the display pane 306. As shown in Fig. 17, markers 462 and 464 are provided on the circular screen 450, to help the user interpret the orientation of the circular screen 450 on the 3D pane 306 and the on the RAO and LAO views. The marker 262 might be blue and the marker 264 might be red.

The user can set the base direction the navigation pane 116 by operating the "apply" button 452 by pointing at the button with a cursor and clicking, using mouse 74 or 82 or keyboard 80. This sets the current direction as the direction though the center of the screen 450. The user can then specify a direction for the arrow 350 by selecting a point on the screen 450, by pointing with the cursor and clicking, using mouse 74 or 82, or keyboard 80. As shown in Fig. 17, the screen 450 has vertical and horizontal cross hairs 466 and 468, and a plurality of radially extending markers 470, at 30 degree intervals. There are a plurality of concentric circular markers 472 representing regular angular intervals (10 degree intervals in the preferred embodiment), with specified intervals (30 degree intervals in the preferred embodiment) indicated by bold markers 472a. The circular screen 450 actually represents a hemisphere of space, and is represented as such with hemisphere 450' on display 306 in Fig. 17. The hemisphere 450' includes markers 462' and 464' corresponding to the markers 462 and 464 on circular screen 450. The screen 450 allows the user to orient the arrow 350 at a number of points to draw radial and circular lines.

The toolbar 304 preferably also includes an indicator 480, an apply button 482, a reduce button 484, and an angle indicator 486. The indicator 480 allows the user to select among a "manual apply" mode, in which the user must affirmatively apply the selected field, an "automatic" mode in which the selected field direction is automatically applied, and a "locked" mode in which the field cannot be applied without changing the mode to either "manual apply" or "automatic". The apply button 482 and the reduce button 484 are preferably virtual buttons which are operated by pointing the cursor and clicking, for example with mouse 74 or 82, or keyboard 80. Operating the apply button 482 when the interface is not in the automatic or locked modes causes the magnetic navigation system to apply a magnetic field to orient the distal end of the medical device in the orientation of the arrow 350. Operating the reduce button 484 causes the magnetic navigation system to "turn off" the magnetic field. The indicator 486 indicates the angular difference between the previously applied magnetic field (arrow 352) and the desired new orientation (arrow 350). Of course rather than discrete navigation, in which the arrow 350 is successively oriented and the magnetic field applied, the interface could be adapted to operate in a continuous navigation or automatic mode in which the field is automatically applied in the direction of arrow 350.

The interface also includes an advancer control pane 316. The advancer control pane 316 displays the length of extension of the medical device being navigated. The pane 316 has three buttons: a reset zero button 490, a zoom in button 492, and a zoom out button 494. The pane 316 also has three user settable flags 496, 498 and 500, and one system settable flag 502. The user can use the reset zero button 490 to reset the current extension of the medical device as the zero position. The user can advance and retract the medical device using the zoom in and zoom out buttons 494 and 496. The extension of the medical device from its zero position is displayed as a colored bar on the scale 504. The user can set three flags to mark desired locations by operating the virtual buttons 496, 498, and 500. Operating any one of the buttons causes the corresponding flag to appear on the scale 504, and allows the user to name the flag for future reference. In modes where the system automatically calculates the applied magnetic field and extension to reach a particular target, the system displays the path of the device a dashed line, the required field as a green arrow, and the required extension by positioning the system flag 502 on the scale 504. This aids the user in extending or retracting the medical device to the proper position to reach the target.

The interface also includes an information block 318, displaying the version of the software, and including a pick window 506 to allow the user to select the particular type of device being navigated. The properties of the device are then used in calculating and displaying the configuration of the device to reach a selected point, and determining the required magnetic field and device extension to reach the desired point.

### Operation

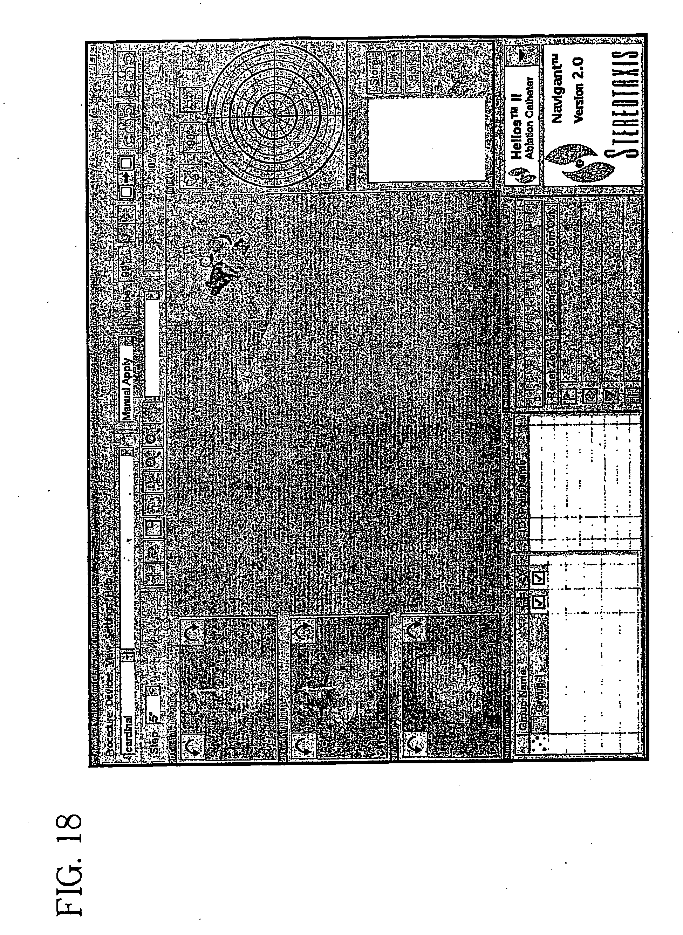

In operation the user can visualize the current direction of the device represented by arrow 352 and the desired new direction for the device represented by arrow 350, on the 3-D pane 306 or on the x-ray images on monitors 86, 88, and 90. The user can selected the orientation of the arrow 350 in a number of ways using panes 308, or 310, or 314, using the menus 328 and 330 on the tool bars 304, or simply selecting a point in the three dimensional display, and allowing the system to calculate the field and direction to reach a selected point 16. See Fig. 18.

The user can select the orientation of arrow 350 (representing the magnetic field to apply) in a variety of ways. On pane 308 the user clicks on buttons 368 and 370, 374 and 376, and 380 and 382, to move the arrow 350 in each of the coronal or frontal plane, the median or saggital plane, and the horizontal or transverse plane to move the arrow. Alternatively, the user can select the orientation of arrow 350 by using the pane 312. The user selects a point on the menu table 408 by pointing and clicking with the cursor to set the orientation of arrow 350 to the orientation associated with point selected. Alternatively, the user can select the orientation of arrow 350 using the pane 312. The user can select a stored orientation by selecting a category on menu 428, and a direction on menu 430. The user can select a user-stored direction by selecting a direction vector from the menu table 436. The user can select a previously used direction by using the buttons 432 and 434 to recall one of the last previously used direction. Finally, the user can select an orientation by picking a point on a screen 450 in pane 314.

Once the direction of the arrow 350 is selected, the navigation system can be operated by operating the apply button 482. This can operate a magnetic navigation system to apply a field in the direction 350, or it can operate a magnetic navigation system to apply a field to cause the medical device to align in the direction 350, either by using feedback of the catheter position or by calculating or using a look-up table to account for the properties of medical device.

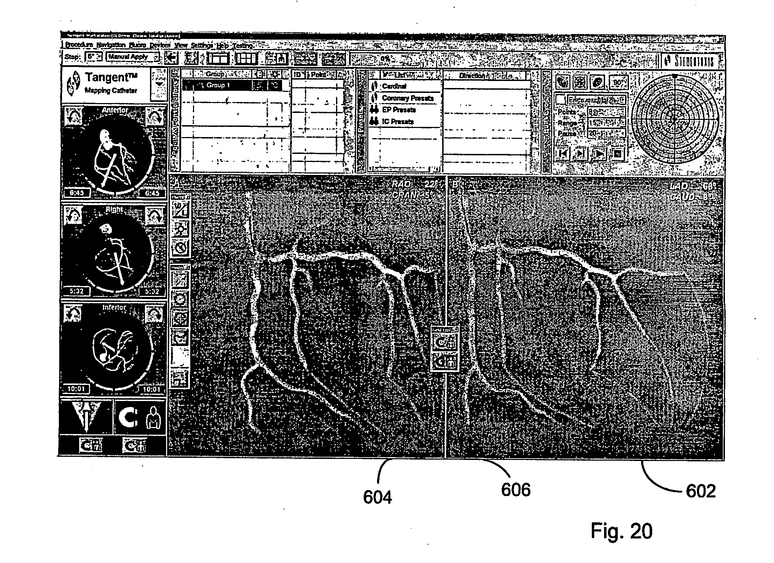

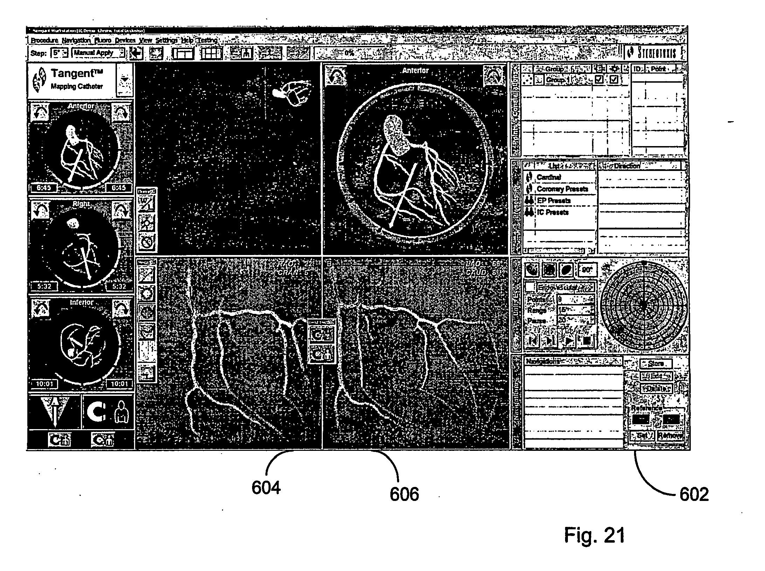

A third embodiment of an interface is illustrated in Figs. 20-26. The interface is adapted for controlling a magnetic navigation system that applies a magnetic field in a selected direction to an operating region in a subject to magnetically orient a medical device in the operating region. The interface comprises a display 602 on which at least one image of the operating region is displayed, and in this preferred embodiment the display has panes 604 and 606 for displaying images of the operating region from two different planes, to facilitate identifying points in three dimensional space in the operating region. The interface further comprises an input device, such as a mouse (not shown) for identifying points in the operating region on the at least one image on the display, for example by moving a cursor or other indicator over the display, and "clicking" on the selected point. Of course the interface could be any other device for identifying points on the display, including joysticks, touch screen displays, light pens, etc. By identifying a point on the image on each of the panes 604 and 606, a user can uniquely identify a point in three-dimensions in the operating region.

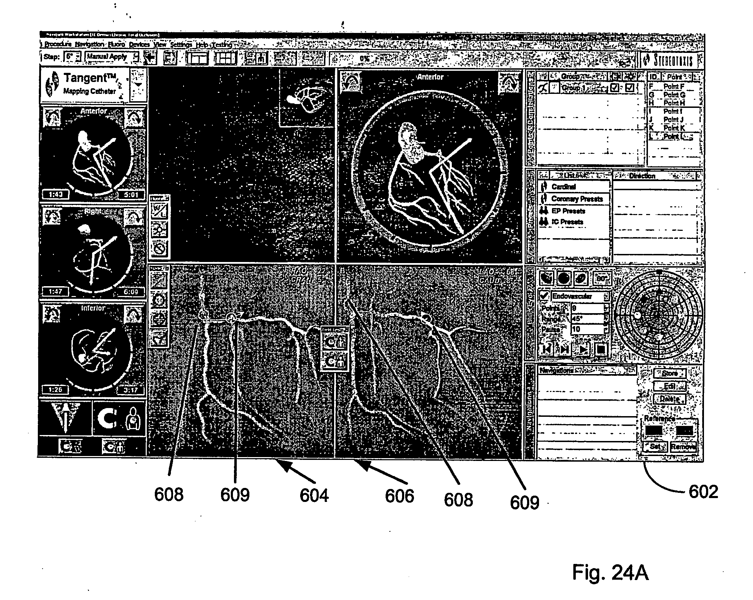

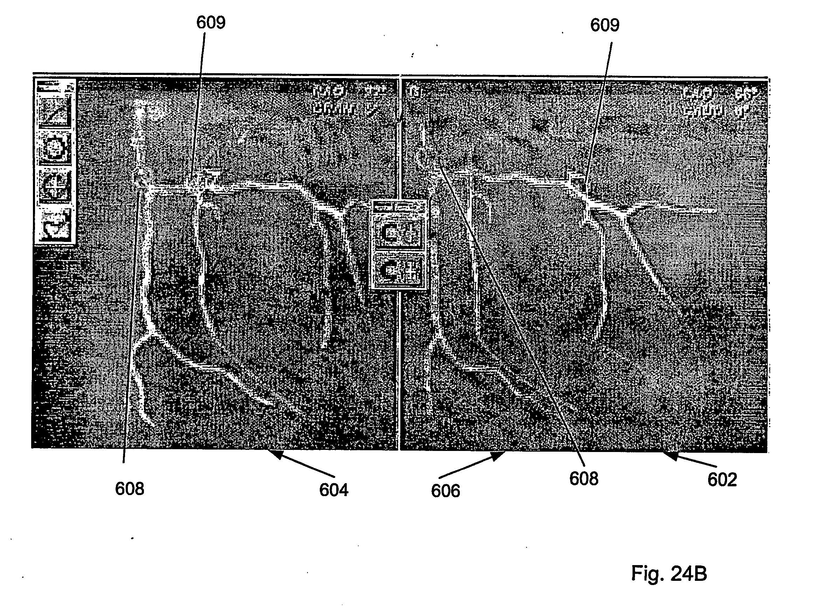

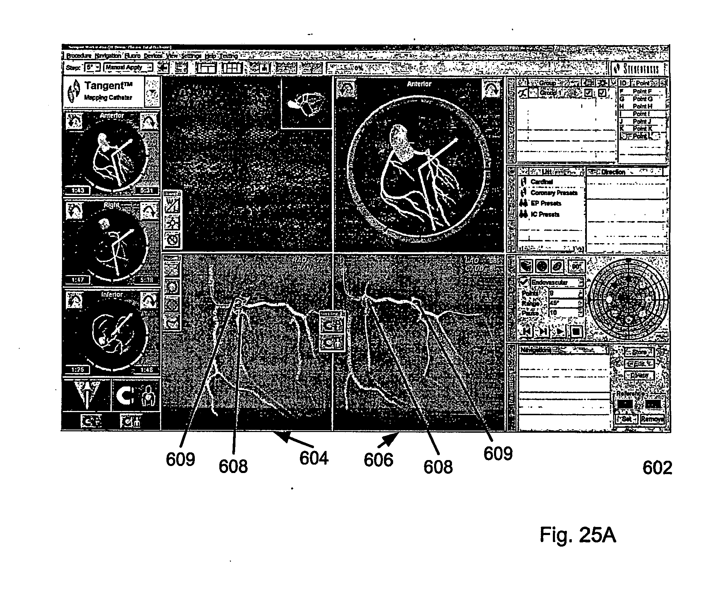

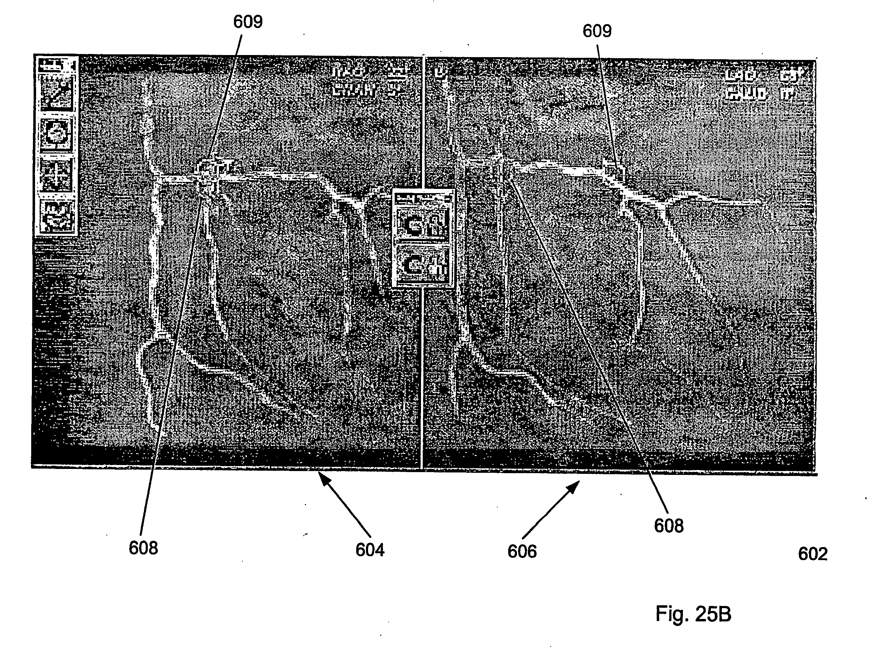

The interface includes a processor that, after the user selects a point in the operating region, determining an application point in the operating region which is on a predetermined branched path through the subject's vasculature and which is closest to the identified point. The interface then determines (e.g., by calculation or use of a reference table) the direction that is tangent to the predetermined branched path at the application point. As shown in Figs. 24 and 25, this direction can be displayed by indicators 608, which may be color coded to distinguish them from indicators 609 of the previously applied direction. The interface, preferably through the processor, then causes the magnetic navigation system to apply a magnetic field at the application point, in a direction tangent to the predetermined path at the application point.

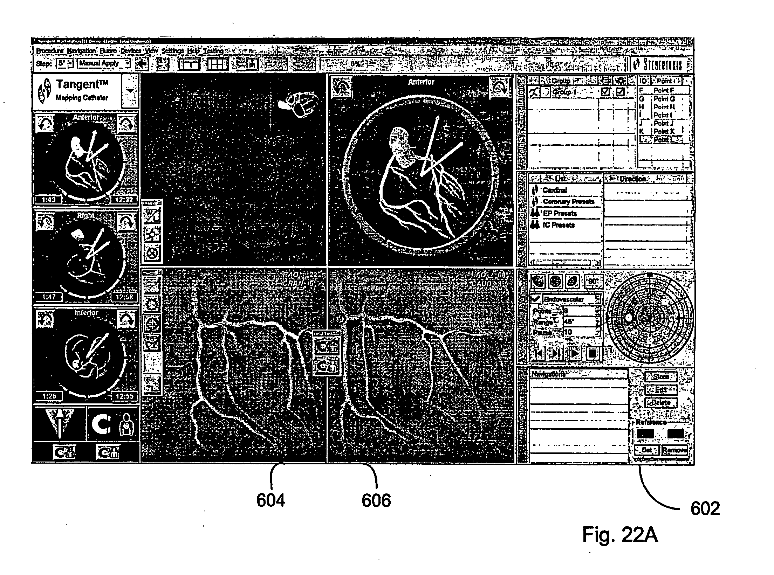

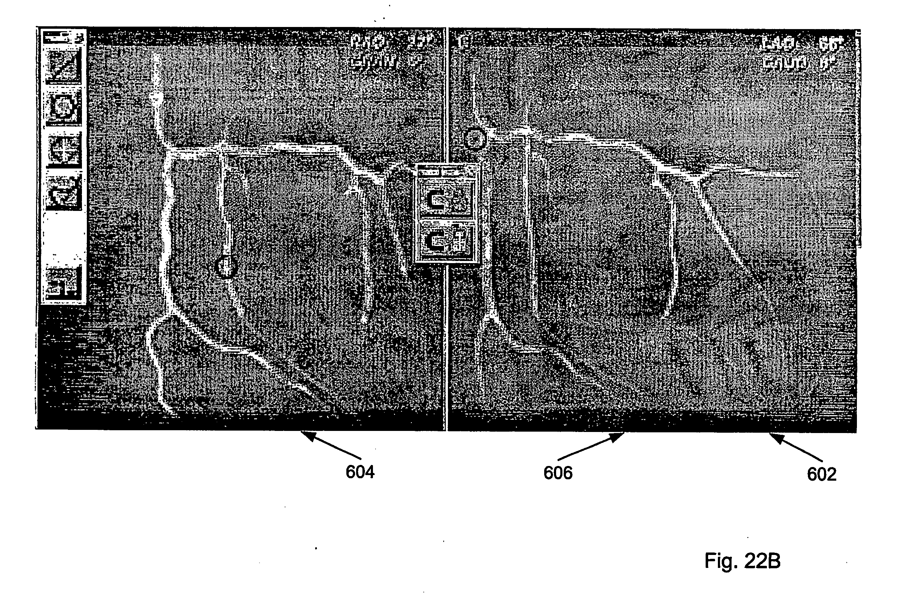

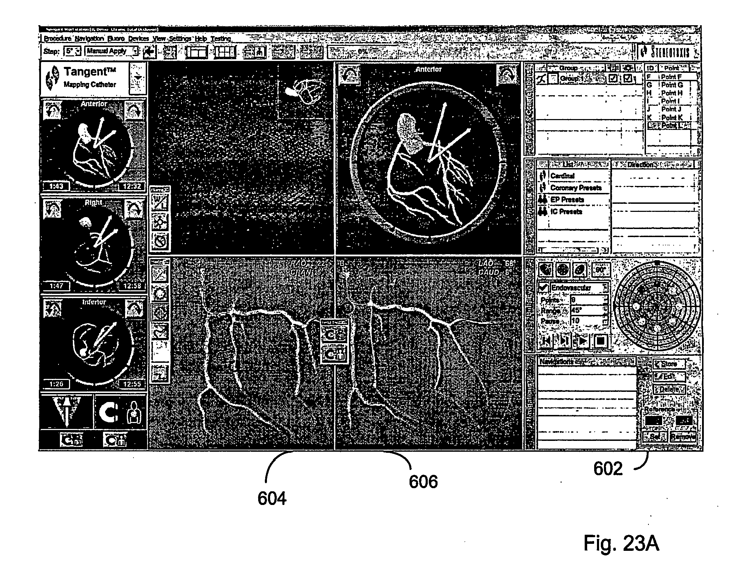

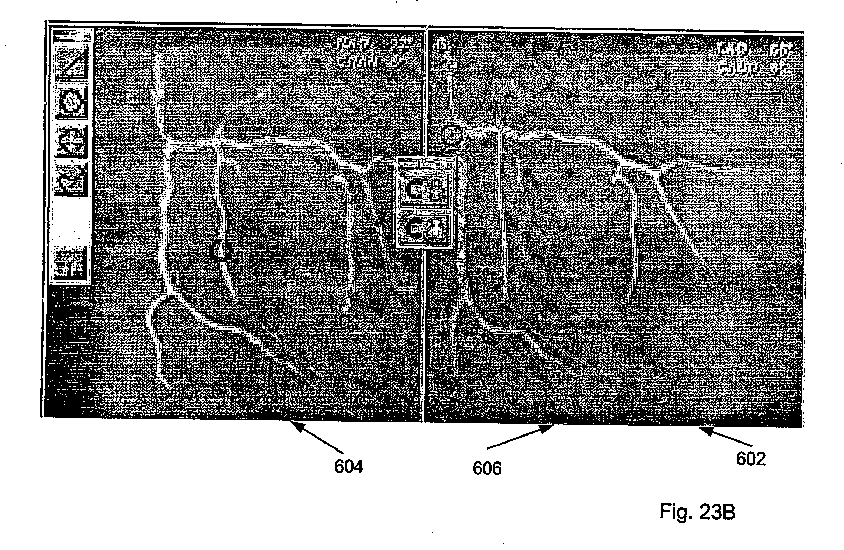

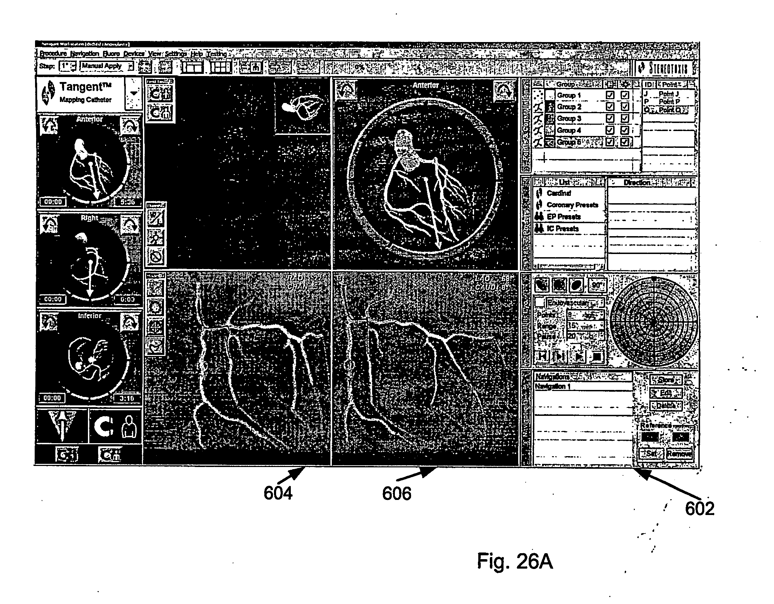

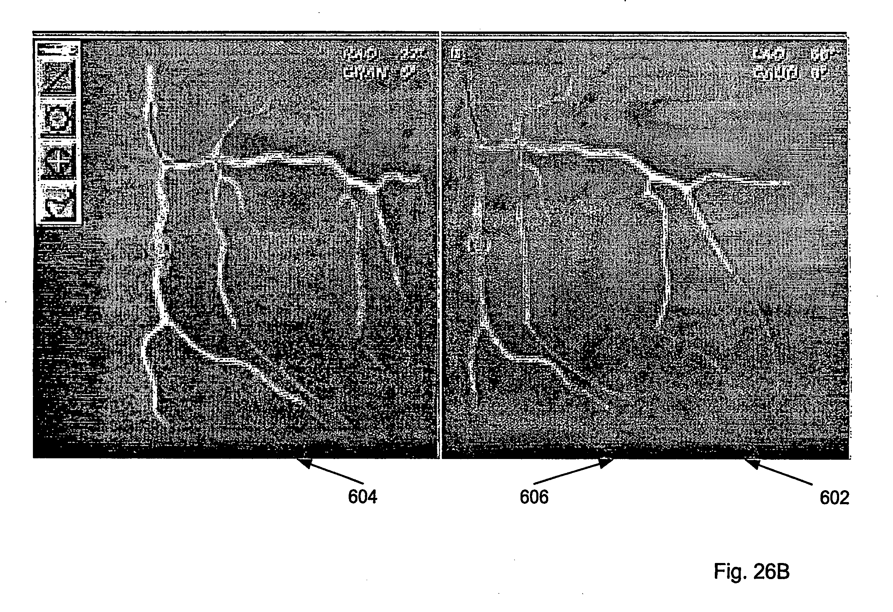

As shown in Figs. 22 and 23, the predetermined branched path can be manually determined prior to beginning the procedure. A user can use the interface to identify a plurality of points on the vasculature in the operating region, uniquely identifying each point in three dimensional space by identifying it on the two panes 604 and 606, using the input device (see Fig. 22). After points have been identified along the vasculature, the processor can automatically connect the points to form the predetermined branched path by connecting each point with its next nearest neighbors (see Fig. 23). The processor then can overlay or superimpose the predetermined branched path over the images of the operating region on the panes 604 and 606, so that the user can verify the accuracy of the branched path, and make adjustments if necessary. As shown in Fig. 26, each branch can be displayed in a different color to help the user visualize the operating region. This is particularly helpful when viewing the operating region in two planes on the panes 604 and 606. Where the vasculature curves, or is branched, the points must be identified fairly closely together, while wherein the vasculature in straight and unbranched, the user does not have to identify as many points. Alternatively, the predetermined branched path can be determined through image processing, which can be assisted by the injection of contrast medium, if necessary.

Thus the processor creates the predetermined branched path through the vasculature in an operating region in a subject's vasculature, by accepting the identification of a plurality of points on the subject's vasculature on at least one image of the operating region; and connecting each point with its nearest neighboring point to form the branched path through the vasculature.

The interface thus can be used to operate a magnetic navigation system to apply a magnetic field in a selected direction in an operating region in a subject, to magnetically orient a medical device in the operating region. The user first identifies a plurality of points along the subject's vasculature in an image of the operating region in the subject. The user then connecting each point to the closest adjacent point to create a network of navigable paths through the subject's vasculature. This can be done manually, but is preferably done automatically by a computer processor. The user then identifies a point where on the image of the operating region, where the user wants to navigate. The computer processor can then determine an application point that is on the previously determined network of navigable paths, closest to the selected point. The computer processor also determines the direction tangent to the network of navigable paths at the application point. The interface then causes the magnetic navigation system to apply magnetic field at the application point in a direction tangent to the navigable path at the application point.

The interface accepts the identification of a selected point on an image of the operating region, determines an application point on a predetermined navigable path through the subject's vasculature in the operating region corresponding that is closest to the selected point; and applies a magnetic field at the application point in a direction tangent to the navigable path at the application point. A magnetic navigation system incorporating the interface may have one or more stationary electromagnetic coils, or one or more movable electromagnets and/or permanent magnets. The interface selectively powers the stationary electromagnets, selectively powers and moves the moveable electromagnets, or selectively moves the permanent magnets to apply the appropriate magnetic field at the operating point in the selected direction.

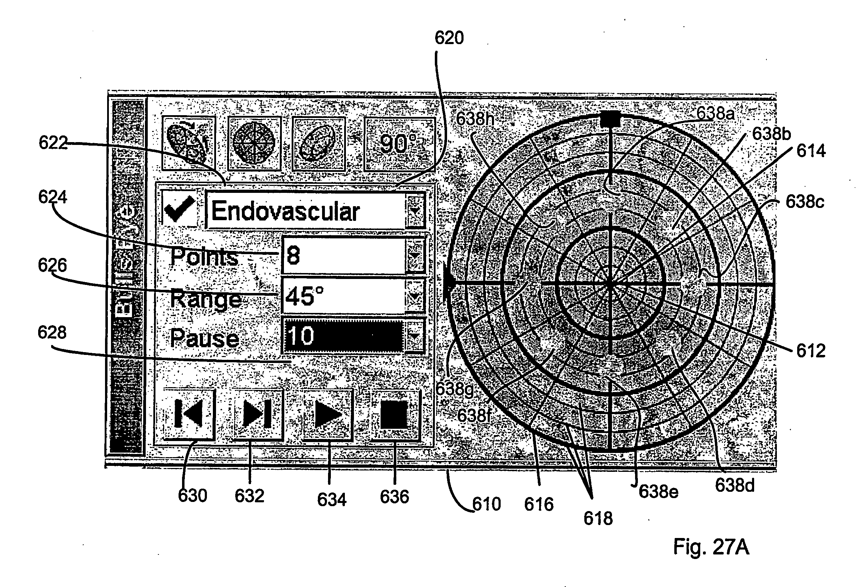

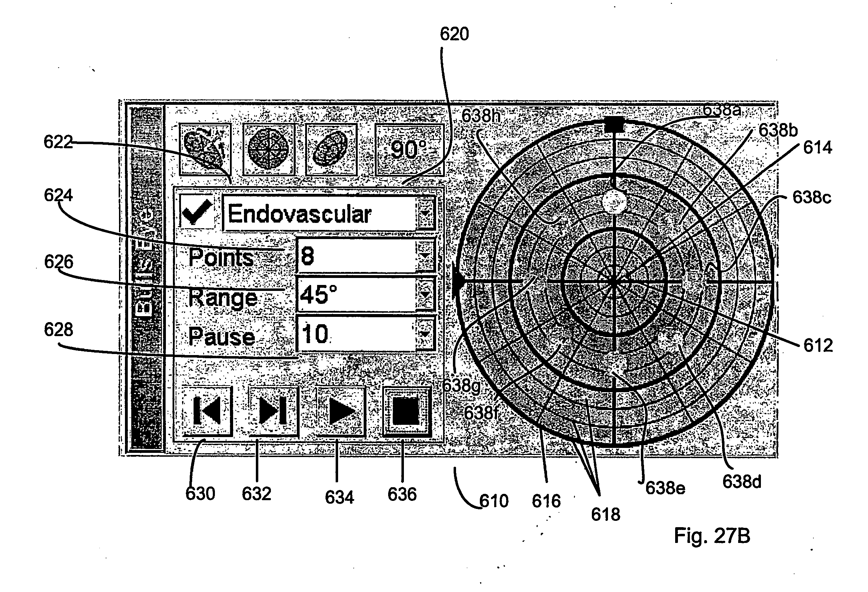

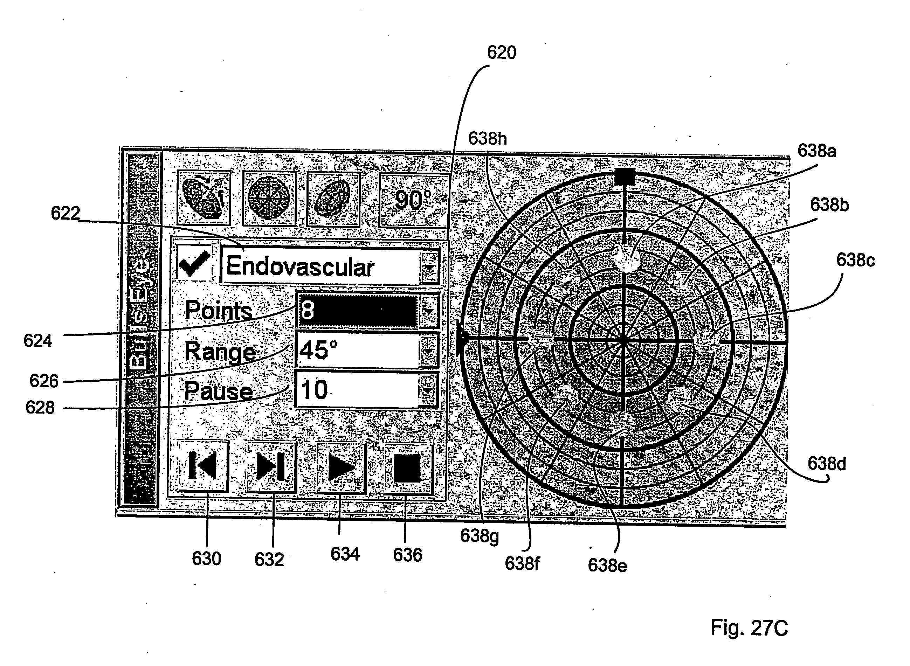

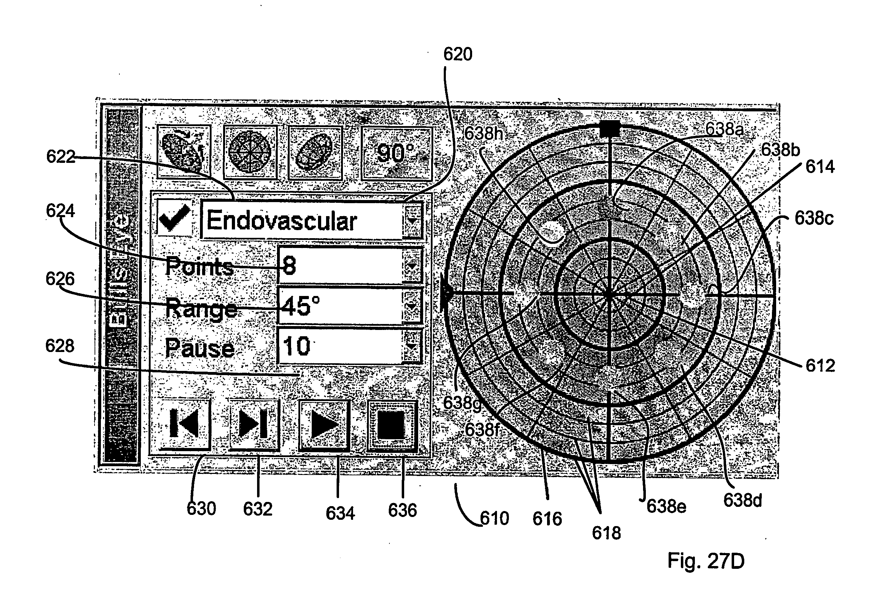

Another control of the interface of the third embodiment is illustrated in Fig. 27. This control operates a magnetic navigation system that applies a magnetic field in a selected direction to an operating region in a subject to magnetically orient a medical device in the operating region. The interface facilitates the specification of the direction of the magnetic field to be applied by the magnetic navigation system, and includes a display pane 610 on which a representation 612 of the current orientation of the medical device (or the currently applied magnetic field) is displayed. In this preferred embodiment the representation 612 is a dot 614 at the center of a circular grid 616 comprising a plurality of concentric circles 618 representing angular deflections from the axis of the medical device. The display pane 610 also includes a selector 620 for selecting one of a plurality of predetermined patterns of new orientations. The interface includes a input device for selecting one of the plurality of patterns of new orientations. This input device may be a mouse and/or a keyboard for operating the selector. Of course, some other input device, such as a joystick, touch screen, etc. could be used for selecting a pattern,

The selector 620 includes a pick box 622 for selecting the type of pattern. In this preferred embodiment there are preferably at least two types of patterns, a circular pattern generally concentric about the current position of the medical device, and a spiral pattern originating at the current position of the medical device. The selector preferably also includes a pick box 624 for selecting the number of new positions in the pattern. The selector preferably also includes a pick box 626 for selecting the angular displacement of the pattern from the current position. The selector may also include a pick box 628 for selecting the delay between movement among the positions in the patter. Lastly, the selector 620 can include a previous position virtual button 630, a next position virtual button 632, a play virtual button 634, and a stop virtual button 636.

The user selects the type of pattern in pick box 622, the number of new positions in the pattern in pick box 624, the angular displacement of the pattern in pick box 626, and if desired a delay time in pick box 628. The selected pattern is displayed on the circular grid 616 as a plurality of dots 638. The user can then operate the magnetic navigation system by clicking on the virtual buttons 630, 632, 634, and 636. Operating button 630 causes the interface to operate the magnetic navigation system to the previous position in the pattern. Operating virtual button 632 causes the interface to operate the magnetic navigation system to the next position in the pattern. Operating the virtual button 634 causes the interface to operate the magnetic navigation system to successively move to each position in the pattern. Operating the virtual button 634 stops automatic operation of the interface.

The colors of the representations of the new positions 638 in the pattern preferably indicate the status of each position. For example, as shown in Fig. 27B, the dots 638b ― 638h are a first color (e.g. light grey), indicating that the medical device has not yet been operated to those positions. The dot 638a is a second color (e.g. yellow), indicating it is the current position of the medical device. As shown in Fig. 27C the dots 638b-638h are in a first color, the dot 638a is a second color and a dot of a third color (e.g. green) indicating the movement of the field appears behind dot 638A. As shown in Fig. 27D, dot 638a is a fourth color (e.g. dark grey) indicating that the medical device has already been navigated to the position, the dot 638h is not the second color, indicating it is the current position of the medical device, and dots 638b-638g are the first color, indicating that the medical device still has not been navigated to these positions.

This pattern navigation, and automated pattern navigation, make it easy to navigate the medical device for selected procedures. For example in mapping procedures, wherein it is desirable to move a mapping catheter to trace an electrical signal, automated movement in a circular or spiral or other pattern facilitates the mapping procedure. Similarly, in ablation procedures, where the user needs to move the tip of an ablation catheter to form a closed loop of ablation, automated movement in a circular or other patter facilitates the ablation procedure.

In operation the user can use the interface to operate a magnetic navigation system to apply a magnetic field in a selected direction in an operating region in a subject, to magnetically orient a medical device in the operating region. The user selects one of a plurality of predetermined patterns of new positions for the medical device using the selector 320 and an input/output device, such as a mouse. The user then simply manually operates the magnetic navigation system to successively orient the medical device in each new position of the pattern by operating virtual button 632 or initiate the system automatically moving from position to position after the predetermined delay by operating virtual button 634.

A magnetic navigation system incorporating the interface may have one or more stationary electromagnetic coils, or one or more movable electromagnets and/or permanent magnets. The interface selectively powers the stationary electromagnets, selectively powers and moves the moveable electromagnets, or selectively moves the permanent magnets to apply the appropriate magnetic field at the operating point in the selected direction.

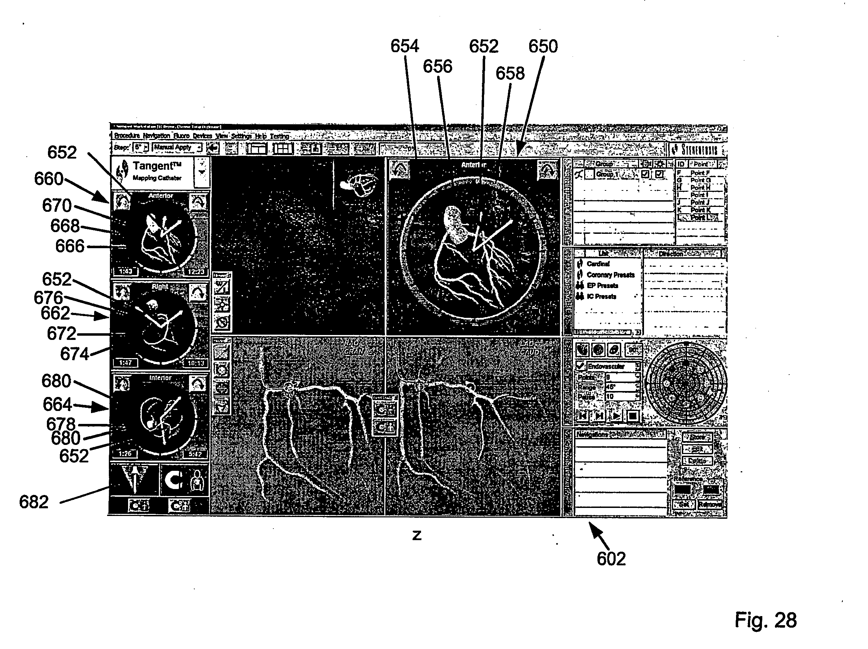

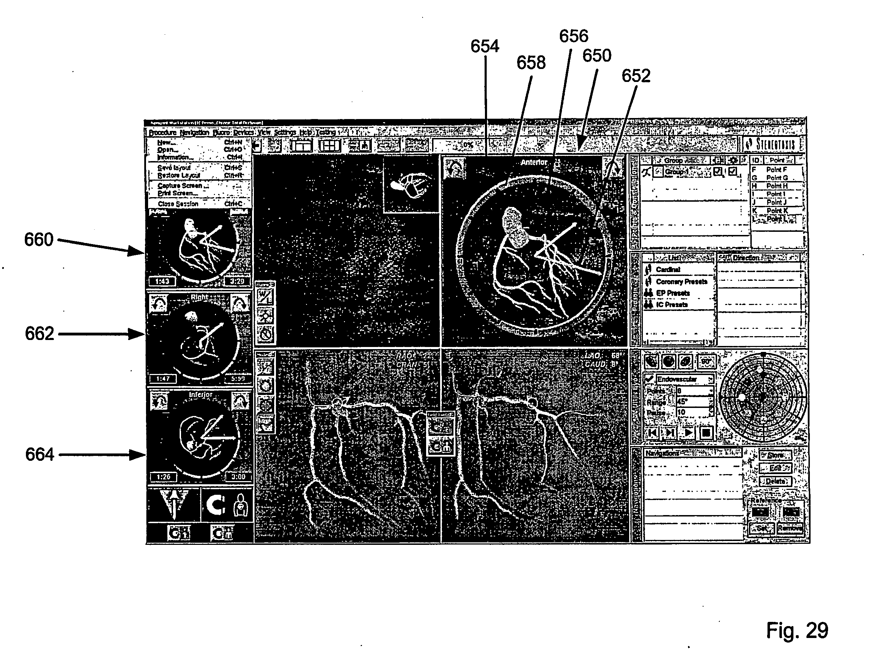

Another control of the interface of the third embodiment is illustrated in Fig. 28 and 29. The control operates a magnetic navigation system that applies a magnetic field in a selected direction to an operating region in a subject to magnetically orient a medical device in the operating region. The control facilitates the specification of the direction in which to orient the medical device/apply a magnetic field.

The control comprises a display pane 650 including an indicator 652 for indicating the desired direction of the medical device and/or applied magnet field on a display. This indicator may be an arrow or other element capable of indicating a three-dimensional direction on a two-dimensional display. The display pane 650 includes at least first and second active areas 654 and 656 for separately controlling the indicator 652. An input device for controls a cursor or other indicator on the display pane to click and drag within one of the two active areas, to change the orientation of the indicator 652. Clicking and dragging in the first active area 654 rotates the indicator 652 about an axis perpendicular to the plane of the display, and clicking and dragging in the second active area 656 flattens in the indicator into the plane of the display, and rotates it about an axis perpendicular to the plane of the display. The input device is preferably a mouse, but could also be a joystick, space ball, touch screen or other device.

The indicator 652 is preferably surrounded by a closed shape, and wherein the first active area 654 outside the closed shape, and wherein the second active area 656 is inside the closed shape. In the preferred embodiment the closed shape is a circle 658 which bounds the maximum extension of the indicator 652. The circle preferably has a plurality of indicia around its circumference, and preferably twelve equally spaced indicia oriented like a clock face, for convenient reference by the users.

In a preferred implementation, the are preferably multiple panes showing the orientation of the indicator 652 from different perspectives. As shown in Figs. 28 and 29, panes 660, 662, and 664 can be provided to provide an image of the indicator from three mutually perpendicular perspectives. Each of the panes allows for rotation of the indicator about an axis perpendicular to its particular plane. This, as described above, allows the user to adjust the orientation of the indicator 652.

The indicator 652 in pane 660 is surrounded by a circular frame 666, defining a first active area 668 outside the frame, and a second active area 670 inside the frame. Clicking and dragging in first active area 668 causes the indicator to rotate about an axis perpendicular to the plane of pane 652, while clicking and dragging in second active area 670 causes the indicator to drop into the plane of the pane 660, and rotate in that plane about an axis perpendicular to the plane of the pane 660.

The indicator 652 in pane 662 is surrounded by a circular frame 672, defining a first active area 674 outside the frame, and a second active area 676 inside the frame. Clicking and dragging in first active area 674 causes the indicator to rotate about an axis perpendicular to the plane of pane 652, while clicking and dragging in second active area 676 causes the indicator to drop into the plane of the pane 662, and rotate in that plane about an axis perpendicular to the plane of the pane 662.

The indicator 652 in pane 664 is surrounded by a circular frame 678, defining a first active area 680 outside the frame, and a second active area 682 inside the frame. Clicking and dragging in first active area 680 causes the indicator to rotate about an axis perpendicular to the plane of pane 664, while clicking and dragging in second active area 680 causes the indicator to drop into the plane of the pane 664, and rotate in that plane about an axis perpendicular to the plane of the pane 664.

In operation the interface is used to control a magnetic navigation system to apply a magnetic field in a selected direction in an operating region in a subject to magnetically orient a medical device in the operating region. The user selects the direction in which to apply a magnetic field by clicking and dragging on one of first and second active areas of a display to rotate an indicator indicating the desired direction. Clicking and dragging on the first active area rotating the indicator about an axis perpendicular to the plane of the display, and clicking and dragging on the second active area collapsing the indicator into the plane of the display, and rotating it about an axis perpendicular to the plane of the display. The user then operates the interface to cause the interface to apply a magnetic field to the operating region in the direction indicated by the indicator.

As shown in Fig. 3 this last control mode of navigation can be applied to specifying the field on the other panes. In this plane mode, a clock like circle is superposed over the indicator of the desired new direction. The indicator can be moved about an axis perpendicular to the clock face by clicking and dragging outside the clock face, or it can be moved in the plane of the clock face, also about an axis perpendicular to the clock face, by clicking and dragging inside the clock face.

## Claims

1. An interface (20) for controlling a magnetic navigation system (58) that applies a magnetic field in a selected direction to an operating region in a subject to magnetically orient a medical device in the operating region, the interface including:
a display (24) on which at least one image of the operating region is displayed, and an input device (26) for identifying a point in the operating region on the at least one image on the display(24); and **characterised by** a processor (22) for determining an application point in the operating region on a predetermined branched path through the subject's vasculature that is closest to the identified point, and causing the magnetic navigation system (58) to apply a magnetic field at the application point, in a direction tangent to the predetermined path at the application point.

2. An interface according to claim 1, wherein there are at least two images of the operating region in different planes on the display (24), and wherein the input device (26) comprises a device for moving an indicator (150) over each image of the operating region and for selecting a point on the image indicated by the indicator (150).

3. An interface according to claim 1 wherein the display (24) displays at least two images of the operating region, and wherein the input device (26) moves a cursor in each of the at least two images to identify a point in the operating region of the subject.

4. An interface according to claim 1 wherein the predetermined branched path was either manually identified or determined by automated processing of an imaging of the operating region.

5. An interface according to claim 1 wherein the predetermined branched path was determined from a plurality of points on the subject's vasculature identified on the at least one image of the operating region on the display (24), wherein, in particular, each point on the subject's vasculature was identified on at least two images of the operating region indifferent planes.

6. An interface according to claim 1 wherein the predetermined branched path is superimposed over the image of the operating region on the display (24), and, in particular, wherein each branch of the predetermined branched path is displayed in a different colour.

7. A magnetic navigation system that applies a magnetic field in a selected direction to an operating region in a subject to magnetically orient a medical device in the operating region, the system comprising at least one magnet, and an interface (20) according to claim1.

8. A magnetic navigation system according to claim 7, wherein there are at least two magnets, and further comprising a movable support for each magnet to change the position and orientation of the each magnet to change the direction of the magnetic field applied to the operating region.

## Patentansprüche

1. Schnittstelle (20) zum Steuern eines magnetischen Navigationssystems (58), das ein Magnetfeld in einer ausgewählten Richtung an einen Arbeitsbereich in einer Person anlegt, um eine medizinische Vorrichtung in dem Arbeitsbereich magnetisch auszurichten, wobei die Schnittstelle Folgendes aufweist:
eine Anzeige (24), auf der mindestens ein Bild des Arbeitsbereichs angezeigt wird, und eine Eingabevorrichtung (26) zum Identifizieren eines Punkts in dem Arbeitsbereich auf dem mindestens einen Bild auf der Anzeige (24); und **gekennzeichnet durch** einen Prozessor (22) zum Bestimmen eines Ansatzpunkts in dem Arbeitsbereich auf einem vorherbestimmten verzweigten Pfad **durch** das Gefäßsystem der Person, der dem identifizierten Punkt am nächsten ist, und Bewirken, dass das magnetische Navigationssystem (58) ein magnetisches Feld an den Ansatzpunkt in einer Richtung, die den vorherbestimmten Pfad an dem Ansatzpunkt tangiert, anlegt.

2. Schnittstelle nach Anspruch 1, wobei mindestens zwei Bilder des Arbeitsbereichs in verschiedenen Ebenen auf der Anzeige (24) vorliegen und wobei die Eingabevorrichtung (26) eine Vorrichtung zum Bewegen eines Anzeigeelements (150) über jedes Bild des Arbeitsbereichs und zum Auswählen eines Punkts auf dem Bild, auf den das Anzeigeelement (150) hinweist, umfasst.

3. Schnittstelle nach Anspruch 1, wobei die Anzeige (24) mindestens zwei Bilder des Arbeitsbereichs anzeigt und wobei die Eingabevorrichtung (26) einen Cursor in jedem der mindestens zwei Bilder bewegt, um einen Punkt in dem Arbeitsbereich der Person zu identifizieren.

4. Schnittstelle nach Anspruch 1, wobei der vorherbestimmte verzweigte Pfad entweder manuell identifiziert oder durch automatisierte Verarbeitung einer Abbildung des Arbeitsbereichs bestimmt wurde.

5. Schnittstelle nach Anspruch 1, wobei der vorherbestimmte verzweigte Pfad aus mehreren Punkten in dem Gefäßsystem der Person bestimmt wurde, die auf dem mindestens einen Bild des Arbeitsbereichs auf der Anzeige (24) identifiziert wurden, wobei insbesondere jeder Punkt in dem Gefäßsystem der Person auf mindestens zwei Bildern des Arbeitsbereichs in verschiedenen Ebenen identifiziert wurde.

6. Schnittstelle nach Anspruch 1, wobei der vorherbestimmte verzweigte Pfad über das Bild des Arbeitsbereichs auf der Anzeige (24) gelegt wird und insbesondere wobei jede Verzweigung des vorherbestimmten verzweigten Pfads in einer anderen Farbe angezeigt wird.

7. Magnetisches Navigationssystem, das ein Magnetfeld in einer ausgewählten Richtung an einen Arbeitsbereich in einer Person anlegt, um eine medizinische Vorrichtung in dem Arbeitsbereich magnetisch auszurichten, wobei das System mindestens einen Magneten und eine Schnittstelle (20) nach Anspruch 1 umfasst.

8. Magnetisches Navigationssystem nach Anspruch 7, wobei es mindestens zwei Magneten gibt, und das weiterhin eine bewegliche Halterung für jeden Magneten umfasst, um die Position und Ausrichtung jedes Magneten zu ändern, um die Richtung des Magnetfelds zu ändern, das an den Arbeitsbereich angelegt wird.

## Revendications

1. Interface (20) pour commander un système de navigation magnétique (58) qui applique un champ magnétique dans une direction sélectionnée à une région opératoire chez un sujet afin d'orienter magnétiquement un dispositif médical dans ladite région opératoire, l'interface comprenant :
un dispositif d'affichage (24) présentant au moins une image de la région opératoire et un dispositif d'entrée (26) pour identifier un point dans la région opératoire sur ladite au moins une image présentée sur le dispositif d'affichage (24) ; et **caractérisée par** un processeur (22) pour déterminer dans la région opératoire, sur une trajectoire ramifiée prédéterminée dans le système vasculaire du sujet, un point d'application qui est le plus proche du point identifié et pour faire appliquer par le système de navigation magnétique (58) un champ magnétique au point d'application, dans une direction tangente à la trajectoire prédéterminée au point d'application.

2. Interface selon la revendication 1, dans laquelle au moins deux images de la région opératoire sont présentées dans des plans différents sur le dispositif d'affichage (24) et dans laquelle le dispositif d'entrée (26) comprend un dispositif permettant de déplacer un indicateur (150) sur chaque image de la région opératoire et de sélectionner sur l'image un point indiqué par l'indicateur (150).

3. Interface selon la revendication 1, dans laquelle le dispositif d'affichage (24) présente au moins deux images de la région opératoire et dans laquelle le dispositif d'entrée (26) déplace un curseur dans chacune desdites au moins deux images afin d'identifier un point dans la région opératoire du sujet.

4. Interface selon la revendication 1, dans laquelle la trajectoire ramifiée prédéterminée a été soit identifiée manuellement, soit déterminée par un traitement automatisé d'une imagerie de la région opératoire.

5. Interface selon la revendication 1, dans laquelle la trajectoire ramifiée prédéterminée a été déterminée à partir d'une pluralité de points sur le système vasculaire du sujet identifiés sur ladite au moins une image de la région opératoire présentée sur le dispositif d'affichage (24), en particulier dans laquelle chaque point sur le système vasculaire du sujet a été identifié sur au moins deux images de la région opératoire dans des plans différents.

6. Interface selon la revendication 1, dans laquelle la trajectoire ramifiée prédéterminée est superposée par dessus l'image de la région opératoire sur le dispositif d'affichage (24) et en particulier dans laquelle chaque branche de la trajectoire ramifiée prédéterminée est présentée dans une couleur différente.

7. Système de navigation magnétique qui applique un champ magnétique dans une direction sélectionnée à une région opératoire chez un sujet afin d'orienter magnétiquement un dispositif médical dans la région opératoire, ledit système comprenant au moins un aimant et une interface (20) selon la revendication 1.

8. Système de navigation magnétique selon la revendication 7, dans lequel il y a au moins deux aimants, et comprenant en outre pour chaque aimant un support mobile permettant de modifier la position et l'orientation de chaque aimant afin de modifier la direction du champ magnétique appliqué à la région opératoire.
